# EUROPEAN PATENT SPECIFICATION

(11) **EP 4 382 144 B1**
(45) Date of publication and mention of the grant of the patent: **08.07.2026**
(21) Application number: 22867433.9
(22) Date of filing: 09.09.2022
(51) Int. Cl.: A61L 27/58, A61L 29/16

(54) **DRUG-ELUTING MEDICAL DEVICE AND PRODUCTION METHOD THEREFOR**
ARZNEIMITTEL FREISETZENDE MEDIZINISCHE VORRICHTUNG UND HERSTELLUNGSVERFAHREN DAFÜR
DISPOSITIF MÉDICAL À ÉLUTION DE MÉDICAMENT ET SA MÉTHODE DE PRODUCTION

(30) Priority: 10.09.2021 JP 2021147558
(43) Date of publication of application: 12.06.2024
(73) Proprietor: TERUMO Kabushiki Kaisha, Tokyo 151-0072 (JP)
(72) Inventor: MATSUSHITA, Shuhei, Ashigarakami-gun, Kanagawa 259-0151 (JP); KUROSAKI, Yasuo, Ashigarakami-gun, Kanagawa 259-0151 (JP)
(74) Representative: Casalonga
(86) International application number: PCT/JP2022/033854
(87) International publication number: WO 2023/038112

(56) References cited:
- WO-A1-2014/118382
- WO-A1-2021/006291
- JP-A- 2015 154 925
- JP-A- 2016 508 776
- JP-A- 2016 513 545
- OBIWELUOZOR FRANCIS O ET AL: "Mussel-inspired elastic interpenetrated network hydrogel as an alternative for anti-thrombotic stent coating membrane", CHEMICAL ENGENEERING JOURNAL, ELSEVIER, AMSTERDAM, NL, vol. 347, 16 April 2018 (2018-04-16), pages 932 - 943, XP085398206, ISSN: 1385-8947, DOI: 10.1016/J.CEJ.2018.04.098
- BRICOUT NICOLAS; CHAI FENG; SOBOCINSKI JONATHAN; HERTAULT ADRIEN; LAURE WILLIAM; UNG ALEXANDRE; WOISEL PATRICE; LYSKAWA JOEL; BLAN: "Immobilisation of an anti-platelet adhesion and anti-thrombotic drug (EP224283) on polydopamine coated vascular stent promoting anti-thrombogenic properties", MATERIALS SCIENCE AND ENGINEERING C, ELSEVIER SCIENCE S.A., CH, vol. 113, 13 April 2020 (2020-04-13), CH , XP086159434, ISSN: 0928-4931, DOI: 10.1016/j.msec.2020.110967

## Description

### Technical Field

The present invention relates to a drug-eluting medical device and a method for producing the same.

### Background Art

As a drug-eluting medical device, a drug-eluting stent (hereinafter, also referred to as "DES") is known. The DES is a device in which a surface of a stent, which is a tubular structure capable of expanding and contracting in the radial direction, is coated with a drug such as an immunosuppressive agent. When a lesion such as a stenosis that has occurred in a body lumen, such as a blood vessel is treated using a DES, the operator percutaneously introduces a catheter having a DES mounted thereon into the body lumen to deliver to the lesion, and expands the DES. The drug applied to the DES infiltrates the lesion, and the drug efficacy is locally exerted at the lesion.

The properties required for the DES include high peeling durability between the drug and the stent and sustained release of drug elution.

With respect to the peeling durability, the drug may be peeled off from the stent surface during mounting, delivery, and expansion, thereby reducing the drug loading amount. In particular, when the drug is peeled off when the DES is in the living body, such as delivery or expansion, the peeled drug or thrombus formation originating from the peeled drug may block the body lumen on the peripheral side, leading to a serious disorder. Therefore, high peeling durability between the drug and the stent is required.

With regard to the sustained release, it is often necessary to maintain the drug efficacy for a long period of time in the treatment of the lesion. For example, when the stent is expanded in the stenotic lesion in the blood vessel, physical stimulation by stent expansion induces a biological reaction such as excessive proliferation of smooth muscle cells and causes restenosis. Such a biological reaction starts immediately after implantation of the stent and reaches a peak in about several months to half a year. In this case, elution of the drug of the DES should last for at least several months or longer. Therefore, elution of the drug is required to be sustained release.

From such a viewpoint, various techniques for supporting a drug using a polymer and coating this on the surface of a stent have been disclosed.

Patent Literature 1 discloses, as a medical device having a hydrophilic coating excellent in peeling durability, a technique for forming, on a substrate, a first coating layer coated with polydopamine, which is applicable to a wide variety of substrates made of metals, ceramics, polymers and the like, and is known as an ideal primer, and a second coating layer coated with a crosslinked copolymer having a hydrophilic function on the first coating layer. Patent Literature 1 discloses that the second coating layer may be configured in the form of a crosslinked copolymer formed via a crosslinking agent, and a functional group in the crosslinked copolymer and polydopamine are covalently bonded, and also discloses that the second coating layer may contain a chemical substance (drug) having a pharmacological activity, such as an antithrombotic drug, a hemostatic agent, an angiogenic inhibitor, an angiogenic agent, an antimicrobial agent, an antiproliferative agent, a proliferative agent, and an anti-inflammatory drug. Patent Literature 1 discloses a stent as an example of a medical device to which this technology is applied.

### Citation List

### Patent Literature

Patent Literature 1: JP 2016-508776 A

### Summary of Invention

### Technical Problem

However, when the technique described in Patent Literature 1 is applied to a stent, it is difficult to satisfy sustained release of the drug, which is a property required for the DES. In "Example 5: Formation of hydrophilic coating containing useful chemical substances" of Patent Literature 1, various drugs are introduced into a hydrophilic coating (second coating layer) by a method including, after forming a hydrophilic coating which is the second coating layer, applying an aqueous solution containing heparin and 1-ethyl-3-(3-dimethylaminopropyl)carbodiimide (EDC); alternately forming a heparin layer and a polyamine layer; applying an aqueous solution containing heparin and NaCL; applying an aqueous solution containing doxorubicin; and applying an ethanol solution of silver carbonate and chlorhexidine. In this case, the drug adheres onto the surface of the second coating layer. When the second coating layer has a porous structure and has a gap into which the drug enters, there is also a possibility that the drug enters the gap. However, in general, the drug has a small bonding force to act on the polymer, and in any of the above cases, the agent is released early. In order to sustainably release the drug, the drug is preferably incorporated into a coating layer having a non-porous structure. Even when the polymer having a hydrophilic function of the second coating layer of Patent Literature 1 is replaced with a polymer such as polylactic acid which is well known as a polymer supporting the drug of the DES, a situation in which sustained release of the drug is difficult does not change.

On the other hand, by forming a covalent bond between the second coating layer and the first coating layer in a state where the drug exists in advance, the drug can be incorporated into the coating layer having a non-porous structure. However, in this case, heat or light necessary for forming a covalent bond cleaves a chemical bond in the drug, or a radical of a polymerization initiator generated in a reaction process causes an oxidation reaction at the cleavage site, thereby generating an analog substance in which the original structure of the drug has been changed. Therefore, when the drug efficacy is lost and the analog substance further causes side effects, the analog substance becomes harmful.

The present invention has been made to solve the above-described problems, and an object of the present invention is to provide a drug-eluting medical device in which drug elution is sustained release and the peeling durability of a coating layer is high, and a method for producing the same.

### Solution to Problem

A drug-eluting medical device that achieves the above object includes: a substrate; a first coating layer having a first polymer obtained by autoxidation polymerization of a dopamine molecule or an analog thereof on the substrate; a second coating layer having a second polymer covalently bonded to the first polymer on the first coating layer; and a third coating layer having a drug and a third polymer supporting the drug on the second coating layer, wherein the second polymer and the third polymer are hydrophobic and form an interpenetrating polymer network structure.

A method for producing a drug-eluting medical device that achieves the above object includes: a first step of applying a first solution containing a dopamine molecule or an analog thereof as a first coating material onto a substrate and polymerizing the first coating material to form a first coating layer having a first polymer; a second step of applying a second solution containing a polymer and/or a monomer as a second coating material onto the first coating layer, and then performing heating or light irradiation to form a second coating layer having a second polymer; and a third step of applying a third solution containing a drug and a polymer as a third coating material onto the second coating layer, and then performing drying to form a third coating layer having a third polymer supporting the drug, wherein the second and third polymers are hydrophobic.

### Advantageous Effects of Invention

The drug-eluting medical device configured as described above and the drug-eluting medical device produced by the production method described above are characterized in that the drug has intended drug efficacy and drug elution is sustained release because the drug is supported by the third polymer while having its original structure; and the coating layer has high peeling durability because the third polymer forms an interpenetrating polymer network structure with the second polymer.

### Brief Description of Drawings

Fig. 1 is a cross-sectional view of a substrate and a coating layer of a drug-eluting medical device according to an embodiment.
Fig. 2 is a TEM image of a cross section of a coating layer (interface between a second coating layer and a third coating layer) of Example.
Fig. 3 is a TEM image of a cross section of a coating layer (interface between a second coating layer and a third coating layer) of Example.
Fig. 4 is a TEM image of a cross section of a coating layer (interface between a second coating layer and a third coating layer) of Comparative Example.

### Description of Embodiments

An embodiment of the present invention will be described below with reference to the drawings. Note that dimensional ratios in the drawings may be exaggerated and different from actual ratios for convenience of description.

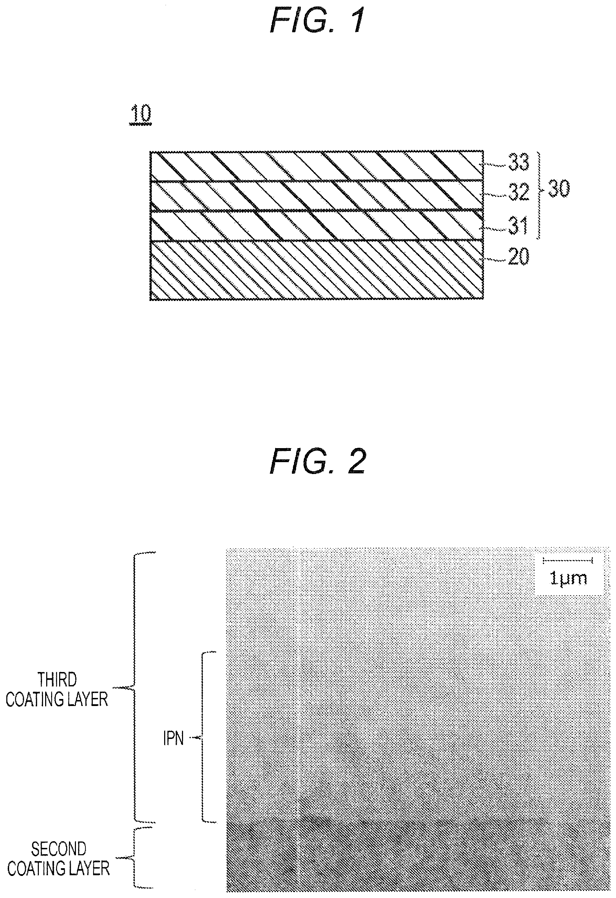

As illustrated in Fig. 1, a drug-eluting medical device 10 according to the present embodiment includes a substrate 20 and a coating layer 30. The coating layer 30 includes a first coating layer 31 that coats the substrate 20, a second coating layer 32 that coats the first coating layer 31, and a third coating layer 33 that coats the second coating layer 32. The first coating layer 31 may coat the entire substrate 20, or may coat at least a part thereof. The second coating layer 32 may coat the entire first coating layer 31, or may coat at least a part thereof. The third coating layer 33 may coat the entire second coating layer 32, or may coat at least a part thereof. When the drug-eluting medical device 10 has a cylindrical shape like the DES, the coating layer 30 may coat all the surfaces of the stent that is the substrate 20 among the outer surface that is the tissue side of the body lumen, the inner surface that is the lumen side of the body lumen, and the side surface located between the outer surface and the inner surface, but may coat only one or two surfaces. Further, the coating layer 30 coats the entire or at least a part of each surface, and the coating layer 30 on each surface may be integrally formed or may be separately formed.

At least a part of the coating layer 30, such as an end of the coating layer 30 in a direction parallel to the surface of the substrate 20, the contact relationship between the substrate 20 and the first coating layer 31, between the first coating layer 31 and the second coating layer 32, or between the second coating layer 32 and the third coating layer 33 is not necessarily be satisfied. For example, the substrate 20 and the second coating layer 32 may be in contact with each other, or the third coating layer 33 is not necessarily provided on the second coating layer 32. The contact relationship between the substrate 20 and the first coating layer 31, the contact relationship between the first coating layer 31 and the second coating layer 32, and the contact relationship between the second coating layer 32 and the third coating layer 33 is only required to be satisfied by at least a part of the coating layer 30.

### <Medical device 10>

The drug-eluting medical device 10 according to the present embodiment is a medical device used for transferring a drug to a body tissue while being in contact with the body tissue. In addition, since drug elution is sustained release, it is suitable for use in a medical device having a long contact time with a body tissue, and more suitable for use in an implantable medical device that can be implanted in the body. Examples of the medical device used for transferring a drug to a body tissue while being in contact with the body tissue include catheters such as balloon catheters, contrast catheters, ablation catheters, suction catheters, perfusion catheters, imaging catheters, and microcatheters, sheaths such as sheath introducers and guiding sheaths, guide wires, and medical patches such as antiphlogistic analgesic patches. Examples of the implantable medical device that can be implanted in the body include stents, stent grafts, artificial blood vessels, artificial bones, artificial heart valves, pacemakers, artificial joints, auxiliary artificial hearts, indwelling catheters, embolic coils, aneurysm clips, thrombus filters, and implantable insulin pumps.

In addition, since the peeling durability of the coating layer is high, a medical device to which a force to peel the coating layer 30 is likely to be applied before and after the drug-eluting medical device 10 according to the present embodiment is introduced to a target position in the body is suitable as the drug-eluting medical device 10 according to the present embodiment. In the implantable medical device that can be implanted in the body described above, a force to peel the coating layer 30 is likely to be applied before and after the medical device is introduced into a target position in the body.

The DES is particularly suitable as the drug-eluting medical device 10 according to the present embodiment because a force to peel the coating layer 30 is likely to be applied at the time of mounting, delivery, and expansion, and elution of a drug that suppresses a biological reaction causing restenosis needs to last for at least several months or more. However, the drug-eluting medical device 10 according to the present embodiment is not limited to the DES, and can also be applied to the above-described medical devices.

### <Substrate 20>

The substrate 20 of the drug-eluting medical device 10 according to the present embodiment is metal, polymer, ceramic, a fibrous protein such as silk or wool. At least a part of the substrate 20 may be composed of one or more of these materials.

Examples of the metal that can be applied to the substrate 20 include, but are not limited to, stainless steel, a cobalt-chromium alloy, platinum, a platinum alloy, a nickel-titanium alloy, a titanium alloy, tantalum, a tantalum alloy, gold, silver, iron, zinc, magnesium, a niobium alloy, and mixtures thereof.

Examples of the polymer that can be applied to the substrate 20 include, but are not limited to, polyglycolic acid, polylactic acid, polycaprolactone, polydioxanone, polytetrafluoroethylene, trimethylene carbonate, polyethylene terephthalate, polybutylene terephthalate, polybutyl methacrylate, polycarbonate urethane, polyether ether ketone, polyolefin, polyester, polyurethane, polyamide, polyether block amide, polyimide, polycarbonate, polyphenylene sulfide, polyphenylene oxide, polyether, silicone, polycarbonate, polymethacrylate, polyvinylpyrrolidone, polyvinyl alcohol, polyvinyl acetate, polyethylene vinyl acetate, polyethylene elastomer, polyvinyl chloride, rubber, silicone rubber, polyhydroxy acid, polyallylamine, polyallyl alcohol, polyacrylamide, polyacrylonitrile, acrylic oxide, polyacrylic acid, polymethacrylic acid, polystyrene, polyoxymethylene, phenol resin, amino epoxy resin, cellulose-based plastic, and copolymers, derivatives, mixtures thereof. The copolymer means any polymer formed from two or more types of monomers. These polymers may or may not be crosslinked. These polymers may be blended with a filler or a colorant.

Examples of the ceramic that can be applied to the substrate 20 include, but are not limited to, silicone oxide, aluminum oxide, silica, hydroxyapatite, glass, calcium oxide, polysilanol, and phosphate.

The surface of the substrate 20 of the drug-eluting medical device 10 according to the present embodiment may be smooth or rough. In addition, in order to enhance adhesion to the first coating layer 31, a known surface treatment technique such as a cleaning treatment or a plasma treatment may be applied to the surface of the substrate 20.

### <First coating layer 31>

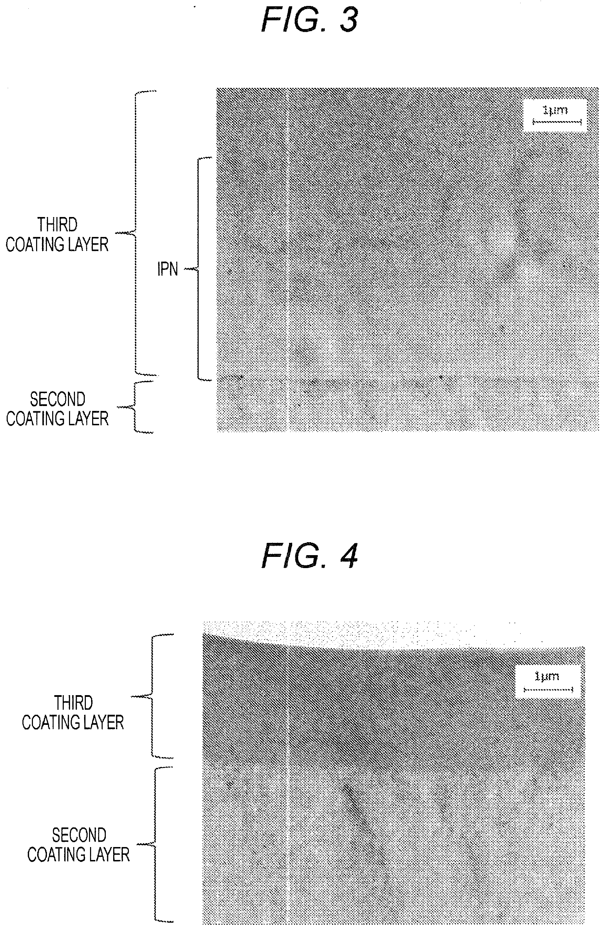

The first coating layer 31 of the drug-eluting medical device 10 according to the present embodiment has a first polymer obtained by autoxidation polymerization of a dopamine molecule or an analog thereof. The first polymer is preferably polydopamine which is a polymer formed by autoxidation polymerization of a dopamine molecule which is catecholamine. In 2007, Lee and Messersmith et al. found that polydopamine can be coated on the surface of a substrate made of a wide range of materials, has high peeling durability through hydrogen bonding and coordinate bonding to a hydrophilic substrate and through a hydrophobic interaction such as a π-π interaction to a hydrophobic substrate, and is also easy to perform secondary modification (Science, 2007, 318, page 426 to 430), and polydopamine has attracted attention as a versatile coating material since this finding. As illustrated in Fig. 3 of Patent Literature 1, a structure in which 5,6-dihydroxyindole or acyclic dopamine generated by oxidation of a dopamine molecule is continuously linked via a covalent bond, and a structure in which a supramolecular assembly is formed by a bond due to a physical interaction other than a covalent bond are proposed. The structure of the first polymer obtained by autoxidation polymerization of a dopamine molecule or an analog thereof in the present invention is not limited to a specific structure, and is all of the structures that can be taken when the dopamine molecule or analog thereof is subjected to autoxidation polymerization.

In addition, the first coating layer 31 has a structure of the first polymer at least in a part thereof. That is, the first coating layer 31 may contain a structure derived from a dopamine molecule or an analog thereof. As an example in which the structure of the first polymer is present in a part of the coating layer, JP 2016-513545 A discloses a coating layer obtained by mixing and polymerizing a dopamine molecule and a molecule covalently bonded to the dopamine molecule in order to improve adhesion between a substrate and polydopamine, and the like. In this case, a part of the coating layer has a polydopamine structure, and a structure derived from a molecule covalently bonded to a dopamine molecule is contained in the coating layer as a structure derived from a molecule other than a dopamine molecule or an analog thereof. As another example, a substance that is not covalently bonded to a dopamine molecule or an analog thereof may be contained in the first coating layer 31. Also in this case, the first coating layer 31 at least partially has a structure of the first polymer, and this case is included in the technical scope of the present invention.

The first coating layer 31 may have a first polymer formed by autoxidation polymerization of a dopamine analog which is an analog of a dopamine molecule. The first coating layer 31 may have a first polymer formed by autoxidation polymerization of a plurality of dopamine analogs. The first coating layer 31 may have a first polymer formed by autoxidation polymerization of one dopamine molecule and two or more dopamine analogs. The chemical formula of the dopamine analog is, for example, the chemical formulas described in paragraphs "0189" to "0193" and "0228" to "0230" of Patent Literature 1.

The thickness of the first coating layer 31 is not particularly limited, but is, for example, 1 to 200 nm, preferably 5 to 150 nm, more preferably 10 to 100 nm, and still more preferably 15 to 80 nm. The thickness of the first coating layer 31 may be uniform or non-uniform. The surface of the first coating layer 31 may be smooth or rough. The thickness of the first coating layer is measured by, for example, an atomic force microscope (AFM).

### <Second coating layer 32>

The second coating layer 32 of the drug-eluting medical device 10 according to the present embodiment has a second polymer which is covalently bonded to the first polymer obtained by autoxidation polymerization of a dopamine molecule or an analog thereof. The second coating layer 32 is formed, for example, by placing a material of the second polymer on the first coating layer 31 and applying energy such as heat or light. The second coating layer 32 may also contain a polymerization initiator. When the second coating layer 32 is formed, a covalent bond is formed between the first polymer and the second polymer.

The covalent bond between the first polymer and the second polymer is formed by, for example, a functional group of the first polymer from which a drawable hydrogen atom on the surface of the first coating layer 31 has been drawn, and a functional group of the second polymer in the second coating layer 32 in contact with the surface of the first coating layer 31. In another example, the covalent bond is formed by a functional group of the polymerizable first polymer on the surface of the first coating layer 31 and a functional group of the second polymer on the second coating layer 32 in contact with the surface of the first coating layer 31. The functional group forming a covalent bond is determined by, for example, the materials and structures of the first polymer and the second polymer. Patent Literature 1 suggests that, when the radical initiator as a polymerization initiator is Norrish type II, the functional group forming a covalent bond on the substrate side is a functional group from which a drawable hydrogen atom on the substrate surface has been drawn, and when the radical initiator is Norrish type I, the functional group forming a covalent bond on the substrate side is a polymerizable functional group on the substrate surface. In the present invention, the mechanism of the covalent bond between the first polymer and the second polymer is not particularly limited.

The second polymer of the second coating layer 32 is preferably a crosslinked polymer obtained by crosslinking a base polymer and a crosslinkable monomer. With such a configuration, a covalent bond is formed between the functional group of the crosslinkable monomer and the first polymer on the surface of the first coating layer 31, and the bonding force between the first coating layer 31 and the second coating layer 32 is improved, so that the peeling durability of the coating layer 30 is improved. In this case, the functional group of the base polymer does not necessarily need to be covalently bonded to the first polymer on the surface of the first coating layer 31, and thus a wide variety of polymers can be selected as the base polymer. A covalent bond may be formed between the functional group of the base polymer and the first polymer on the surface of the first coating layer 31. In addition, a covalent bond may be formed between the functional group of the crosslinkable monomer and the functional group of the base polymer and the first polymer on the surface of the first coating layer.

When the second polymer of the second coating layer 32 is a crosslinked polymer obtained by crosslinking a base polymer and a crosslinkable monomer, examples of the base polymer include polyesters, aliphatic polyesters, polyacid anhydrides, polyorthoesters, polycarbonates, polyphosphazenes, polyphosphate esters, polyvinyl alcohols, polypeptides, polysaccharides, proteins, cellulose, and copolymers, derivatives, and mixtures thereof. Specific examples of the aliphatic polyester include polylactic acid, polycaprolactone, polyglycolic acid, polydioxanone, polybutyrolactone, polyvalerolactone, polyhydroxybutyric acid, polytrimethylene carbonate, and copolymers, derivatives, or mixtures thereof. Examples of the form of the copolymer include, but are not limited to, an alternating copolymer, a random copolymer, a block copolymer, and a graft copolymer.

The monomers constituting these polymers may have optical isomers, but the polymer is not limited to polymers composed of specific optical isomers. For example, lactic acid (LA) which is a constituent monomer of polylactic acid (PLA) includes L-lactic acid and D-lactic acid having different optical activities. Therefore, polylactic acid includes poly-L-lactic acid (PLLA) obtained by polymerizing L-lactic acid, poly-D-lactic acid (PDLA) obtained by polymerizing D-lactic acid, and poly-D,L-lactic acid (PDLLA) which is a random copolymer of L-lactic acid and D-lactic acid, but the present invention is not limited thereto.

In the present invention, a polymer polymerized using lactic acid as a monomer is referred to as a polymer having a lactic acid monomer unit regardless of the optical activity of lactic acid to be used. The lactic acid monomer unit refers to a form in which lactic acid in the polymer is reacted. Polymers polymerized using not only polylactic acid but also lactic acid and other monomers are also included in the polymer having a lactic acid monomer unit.

In addition, proteins such as collagen, fibrin, and elastin, which are natural polymer materials, extracellular matrix components, other biological agents and derivatives thereof, or mixtures thereof may be used as the base polymer.

In the present invention, in a preferred embodiment, the base polymer has a lactic acid monomer unit. That is, the second polymer preferably has a lactic acid monomer unit. The content of the lactic acid monomer unit in the base polymer is preferably 50 mol% or more (upper limit: 100 mol%), and more preferably 70 mol% or more (upper limit: 100 mol%) with respect to all monomers constituting the base polymer. In addition, the second polymer is hydrophobic from the viewpoint of further exhibiting the desired effect of the present invention. Therefore, the base polymer is also preferably hydrophobic.

The weight average molecular weight of the base polymer is preferably 100,000 to 1,000,000, and more preferably 150,000 to 800,000 from the viewpoint of peeling durability and the like. In the present specification, the weight average molecular weight is a value measured under the following measurement conditions by gel permeation chromatography (GPC) using polystyrene as a reference material.

### (Measurement conditions of molecular weight)

Apparatus: Semi-micro GPC system LC-VP system (manufactured by Shimadzu Corporation)
Detector: Shodex (registered trademark) RI-104 (manufactured by Showa Denko K.K.)
Column: Shodex (registered trademark) GPC LF-804 (manufactured by Showa Denko K.K.), two columns used
Guard column: Shodex (registered trademark) LF-G (manufactured by Showa Denko K.K.)
Column temperature: 40°C
Mobile phase solvent: CHCl₃
Flow rate: 1.00 mL/min
Injection volume: 200 µL.

When the second polymer of the second coating layer 32 is a crosslinked polymer obtained by crosslinking a base polymer and a crosslinkable monomer, the crosslinkable monomer is preferably one which has high affinity with the base polymer, and has strong bonding with the first polymer on the surface of the first coating layer 31.

Examples of the crosslinkable monomer include crosslinkable monomers having a functional group such as a vinyl group (CH₂=CH-), an allyl group (CH₂=CH-CH₂-), an acryloyl group (CH₂=CH-CO-), a methacryloyl group (CH₂=C(CH₃)-CO-), or an acrylamide group (CH₂=CH-CO-NH-), which is one type of the vinyl group. In general, the reactivity of the crosslinkable monomer increases as the polarity of the chemical structure connected to the end structure having an unsaturated bond of a functional group (in the above example, CH₂=CH- or CH₂=C(CH₃)-) increases. Since the magnitude relationship of the polarity is (allyl group) < (acryloyl group) ≈ (methacryloyl group) < (acrylamide group), the reactivity of the crosslinkable monomer having an allyl group is often smaller than the reactivity of the crosslinkable monomer having an acryloyl group or a methacryloyl group, and the reactivity of the crosslinkable monomer having an acrylamide group is often larger than the reactivity of the crosslinkable monomer having an acryloyl group or a methacryloyl group. The reactivity of the crosslinkable monomer having a methacryloyl group is slightly smaller than the reactivity of the crosslinkable monomer having an acryloyl group because CH₃ in the methacryloyl group can be steric hindrance, but is equivalent to the reactivity of the crosslinkable monomer having an acryloyl group. When the number of functional groups is different in the crosslinkable monomer having the same type of functional group, the reactivity increases as the number of functional groups increases. Hereinafter, the term " (meth)acrylate" means both acrylate which is a crosslinkable monomer having an acryloyl group and methacrylate which is a crosslinkable monomer having a methacryloyl(methacryloyl) group.

Examples of the bifunctional (meth)acrylate include diethylene glycol di(meth)acrylate, 1,4-butanediol di(meth)acrylate, 1,3-butylene glycol di(meth)acrylate, dicyclopentanyl di(meth)acrylate, glycerol di(meth)acrylate, 1,6-hexanediol di(meth)acrylate, neopentyl glycol di(meth)acrylate, tetraethylene glycol di(meth)acrylate, ethylene glycol di(meth)acrylate, polyethylene glycol di(meth)acrylate, diethylene glycol di(meth)acrylate, triethylene glycol di(meth)acrylate, 1,9-nonanediol di(meth)acrylate, and 1,10-decanediol di(meth)acrylate. Examples of the trifunctional (meth)acrylate include trimethylolpropane tri(meth)acrylate, pentaerythritol tri(meth)acrylate, and tetramethylolmethane (meth)acrylate. Examples of the tetra- or higher functional (meth)acrylate include pentaerythritol tetra(meth)acrylate, ditrimethylolpropane tetra(meth)acrylate, dipentaerythritol penta/hexa(meth)acrylate, dipentaerythritol hexa(meth)acrylate, and dipentaerythritol monohydroxypenta(meth)acrylate. Examples of the crosslinkable monomer having an acrylamide group include N,N'-methylenebis(meth)acrylamide, N,N'-ethylenebis(meth)acrylamide, N,N'-hexamethylenebis(meth)acrylamide, N,N'-benzylidenebis(meth)acrylamide, N,N'-bis((meth)acrylamidomethylene)urea, N-[tris(3-(meth)acrylamidopropoxymethyl)methyl](meth)acrylamide (for example, FOM-03006; N-[tris(3-acrylamidopropoxymethyl)methyl]acrylamide), N,N-bis(2-(meth)acrylamidoethyl) (meth)acrylamide (for example, FOM-03007; N,N-bis(2-acrylamidoethyl)acrylamide), N,N'-[oxybis(2,1-ethanediyloxy-3,1-propanediyl)]bis(meth)acrylamide (for example, FOM-03008; N,N'-[oxybis(2,1-ethanediyloxy-3,1-propanediyl)]bisacrylamide), and N,N'-1,2-ethanediylbis{N-[2-((meth)acryloylamino)ethyl] (meth)acrylamide} (for example, FOM-03009; N,N'-1,2-ethanediylbis{N-[2-(acryloylamino)ethyl]acrylamide}). Examples of the crosslinkable monomer having an allyl group include triallyl trimellitate, triallyl pyromellitate, diallyl oxalate, triallyl cyanurate, and triallyl isocyanurate (TAIC). Among them, the crosslinkable monomer is preferably a (meth)acrylate and more preferably a tetra- or higher functional (meth)acrylate from the viewpoint of having high bondability to the first polymer on the surface of the first coating layer 31.

In the present invention, the base polymer preferably has a lactic acid monomer unit, and the crosslinkable monomer is preferably a (meth)acrylate from the viewpoint of high affinity between the base polymer and the crosslinkable monomer.

When the second polymer is a crosslinkable polymer obtained by crosslinking a crosslinkable monomer and a base polymer, the content ratio (weight ratio) between the crosslinkable monomer and the base polymer is not particularly limited, but the crosslinkable monomer is contained preferably in an amount of 1 wt% or more and 95 wt% or less, more preferably in an amount of 5 wt% or more and 90 wt% or less, still more preferably in an amount of 10 wt% or more and 90 wt% or less, still even more preferably in an amount of 30 wt% or more and 90 wt% or less, and particularly preferably in an amount of 30 wt% or more and 70 wt% or less with respect to the weight (100 wt%) of the base polymer. In an embodiment, the crosslinkable monomer is contained in an amount of preferably 20 wt% or more and 80 wt% or less, more preferably 30 wt% or more and 75 wt% or less with respect to the weight (100 wt%) of the base polymer. When the crosslinkable monomer is contained within the above range, an interpenetrating polymer network structure described later can be efficiently formed, and the desired effect of the present invention can be further exhibited.

The crosslinkable monomer in the present invention is not limited to those having a vinyl group, an allyl group, an acryloyl group, a methacryloyl group, or an acrylamide group as a functional group. Examples of the crosslinkable monomer having other functional groups include maleimide-based compounds such as N-phenylmaleimide and N,N'-m-phenylenebismaleimide, compounds having two or more triple bonds, such as dipropargyl phthalate and dipropargyl maleate, and divinylbenzene.

The crosslinked polymer may be formed by crosslinking a base polymer and a crosslinkable monomer, or may be formed by crosslinking a constituent monomer of a base polymer and a crosslinkable monomer. The structure of the crosslinked polymer formed by crosslinking a constituent monomer of a base polymer and a crosslinkable monomer also has a structure as a crosslinked polymer obtained by crosslinking a base polymer and a crosslinkable monomer. The crosslinked polymer obtained by crosslinking a base polymer and a crosslinkable monomer is a polymer having a constituent monomer unit of the base polymer and a crosslinkable monomer unit in the molecular structure of the polymer. Here, the constituent monomer unit of the base polymer refers to a form in which the constituent monomer of the base polymer in the crosslinked polymer is reacted, and the crosslinkable monomer unit refers to a form in which the crosslinkable monomer in the crosslinked polymer is reacted.

The crosslinked polymer may be composed of one type of base polymer or one type of constituent monomer of the base polymer, and one type of crosslinkable monomer, but may be composed of a plurality of base polymers or a plurality of constituent monomers of the base polymer, and a plurality of crosslinkable monomers.

The second polymer of the second coating layer 32 need not have a structure in which a base polymer and a crosslinkable monomer are crosslinked. For example, when a polymer having any one functional group of a vinyl group, an allyl group, an acryloyl group, a methacryloyl group, and an acrylamide group or a plurality of functional groups selected therefrom is used as the second polymer, this is placed on the first coating layer 31, and energy such as heat or light is applied to form the second coating layer 32, a covalent bond may be formed between the first polymer and the second polymer. The number and positions of these functional groups in the second polymer are not limited. In this case, the type of the functional group of the second polymer is not limited to the vinyl group, the allyl group, the acryloyl group, the methacryloyl group, and the acrylamide group, and is all the functional groups that can form a covalent bond with the first polymer.

The second coating layer 32 is only required to contain the second polymer covalently bonded to the first polymer at least in a part thereof, and may contain an additive other than the second polymer. At the time of forming the second coating layer 32, an additive intentionally added or unintentionally included may form a part of the second polymer in a covalently bonded form.

The second coating layer 32 may contain a polymerization initiator. As the polymerization initiator, a thermal polymerization initiator and a photopolymerization initiator are known, but the polymerization initiator is not limited to either one. In addition, a plurality of polymerization initiators may be used. Examples of the photopolymerization initiator include alkylphenone-based photopolymerization initiators such as benzyldimethylketal, α-hydroxyalkylphenone, α-aminoalkylphenone, and 2-hydroxy-1-[4-(2-hydroxyethoxy)phenyl]-2-methyl-1-propanone, acylphosphine oxide-based photopolymerization initiators such as MAPO and BAPO, and oxime ester-based photopolymerization initiators. A commercially available polymerization initiator may also be used. Examples of the commercially available photopolymerization initiator include Irgacure 2959, 184, 1173, 907, 369E, 379EG, TPO, and 819 manufactured by BASF SE. Although the form of the polymerization initiator may be changed after energy is applied, the form of the polymerization initiator present in the second coating layer 32 may be the changed form.

The crosslinking mechanism and the polymerization mechanism of the second polymer are not limited. In addition, the primary structure, the secondary structure, and the higher order structure of the second polymer are not limited.

The thickness of the second coating layer 32 is not particularly limited, but is 1 to 500 nm, preferably 5 to 400 nm, more preferably 10 to 350 nm, and still more preferably 30 to 250 nm. The thickness of the second coating layer 32 may be uniform or non-uniform. The surface of the second coating layer 32 may be smooth or rough. The thickness of the second coating layer is measured by, for example, an AFM.

### <Third coating layer 33>

The third coating layer 33 of the drug-eluting medical device 10 according to the present embodiment has a drug and a third polymer supporting the drug on the second coating layer 32. The third polymer in the third coating layer 33 forms an interpenetrating polymer network structure with the second polymer in the second coating layer 32.

The interpenetrating polymer network structure is also referred to as an interpenetrating polymer network (IPN), and means a structure in which one polymer network and another polymer network are entangled with each other without a covalent bond therebetween. When the interpenetrating polymer network structure is formed, the bonding force is improved in each polymer network. Therefore, it is difficult to separate the entire polymer network after formation into individual polymer networks before formation. In the present invention, the third polymer in the third coating layer 33 and the second polymer in the second coating layer 32 form an interpenetrating polymer network structure, whereby the bonding force between the third coating layer 33 and the second coating layer 32 is improved, and the peeling durability of the coating layer 30 is improved.

The formation of the interpenetrating polymer network structure can be confirmed, for example, by observing a cross section including an interface of each layer by a transmission electron microscope (TEM). For example, a sample in which a cross section of the coating layer 30 is exposed can be obtained by embedding the coating layer 30 of the present invention in a resin, and slicing the sample embedded in a resin with an ultramicrotome (Leica EM UC7). The interpenetrating polymer network structure formed between the second coating layer 32 and the third coating layer 33 can be confirmed by TEM observation of this sample. As the embedding resin, a known resin can be used, and for example, an epoxy resin (Epon812), caprolactone (EVONIK C212), or the like can be preferably used. Specifically, the TEM observation can be performed by the method described in Examples. Here, when an interpenetrating polymer network structure is formed, as a TEM image, in the cross section of the coating layer 30, the interface between the coating layer 32 and the coating layer 33 may be unclear, the lightness of the interface may be uneven, and the lightness of the interface may be different from the lightness of the coating layer 32 and the coating layer 33. Therefore, when the above findings are obtained at the interface between the coating layer 32 and the coating layer 33 in the TEM image, it can be determined that the interpenetrating polymer network structure is formed.

Alternatively, the formation of the interpenetrating polymer network structure can also be determined by analyzing the layer structure using an optical analysis device such as Nano-FTIR. In addition, in a medical device having a coating layer, in order to confirm whether an interpenetrating polymer network structure is formed in the coating layer, a cross section of the coating layer of the medical device may be directly confirmed, or components contained in the coating layer may be identified, and a cross section of a sample of a coating layer prepared with the identified components may be confirmed.

In the present invention, when the second polymer and the third polymer form an interpenetrating polymer network structure, the interface between the second coating layer and the third coating layer becomes unclear, and thus, for example, the cross section of the coating layer can be observed as a four-layer structure of "first coating layer + second coating layer + interpenetrating polymer network structure + third coating layer" or a two-layer structure of "first coating layer + interpenetrating polymer network structure". The present invention may have either form as long as the interpenetrating polymer network structure exists, and the effects of the present invention are exerted in either form.

Here, at the time of preparing the coating layer 30, whether or not the third polymer and the second polymer form an interpenetrating polymer network structure can be visually confirmed, for example. Specifically, in the present embodiment, when a third solution containing the third polymer is applied to the second coating layer 32 containing the second polymer, the third solution containing the third polymer penetrates into the second coating layer, and the second coating layer swells. Since the thickness of the second coating layer changes at this time, the color tone derived from the interference color of the coating film changes. Thereby, it can be determined that the third polymer and the second polymer form an interpenetrating polymer network structure. In addition, it is possible to confirm whether or not an interpenetrating polymer network structure is formed also in a peeling durability test performed in Examples described later.

Although the peeling durability of the coating layer 30 is improved as the formation of the interpenetrating polymer network is promoted, the degree of promotion is not limited in the present invention.

In the present invention, the third polymer is hydrophobic. Since the third polymer is hydrophobic, the third polymer can be dissolved in a solvent. As will be described later, in the present invention, a third solution obtained by dissolving the third polymer in a solvent is applied to the second coating layer, and the third solution penetrates into the second coating layer, whereby the second coating layer swells. Thereby, an interpenetrating polymer network structure is formed between the third polymer and the second polymer. Since the third solution also contains a drug at this time, when the third polymer and the second polymer form an interpenetrating polymer network structure, the drug supported on the third polymer may be incorporated into the network structure. In the present invention, when the third polymer is non-porous, sustained release of the drug for a longer period of time becomes possible. Since the second coating layer needs to be swollen by a solvent, the second polymer is also hydrophobic.

It is considered that the hydrophobicity of the third polymer allows the medical device of the present invention to exhibit peeling durability under a use environment. For example, (but not part of the invention) when the third polymer is hydrophilic (for example, polyethylene glycol or the like), the third coating layer and the second coating layer swell under a use environment, the peeling durability of the coating layer cannot be maintained, and the sustained release of the drug is not exhibited. On the other hand, in the medical device of the present invention, since the third polymer and the second polymer are hydrophobic, the third coating layer and the second coating layer do not swell under a use environment, and the peeling durability of the coating layer can be maintained.

Preferably, the second polymer and the third polymer have an identical monomer unit. With such a configuration, the affinity between the second polymer and the third polymer is improved, the formation of the interpenetrating polymer network between the second coating layer 32 and the third coating layer 33 is promoted, and the bonding force between the second coating layer 32 and the third coating layer 33 is increased, so that the peeling durability of the coating layer 30 is improved. Here, the monomer units of the second polymer and the third polymer refer to forms in which constituent monomers of the respective polymers are reacted. The second polymer and the third polymer have an identical monomer unit if the respective polymers are synthesized using the same constituent monomers regardless of optical activity. Also, in a preferred embodiment, the second polymer and the third polymer have an identical monomer unit in at least part of each polymer.

The drug in the third coating layer 33 is, for example, at least one compound selected from the group consisting of immunosuppressive agents, anticancer agents, antibiotics, antirheumatic agents, antithrombotic drugs, HMG-CoA reductase inhibitors, ACE inhibitors, calcium antagonists, antilipemic drugs, integrin inhibitors, antiallergic agents, antioxidants, GPIIb/IIIa antagonists, retinoids, flavonoids, carotenoids, lipid improvers, DNA synthetic inhibitors, tyrosine kinase inhibitors, antiplatelet agents, anti-inflammatory drugs, hemostatic agents, angiogenic inhibitors, angiogenic agents, biological materials, interferons, and NO production promoters.

Examples of the immunosuppressive agent include sirolimus, everolimus, pimecrolimus, zotarolimus, biolimus, tacrolimus, azathioprine, cyclosporine, cyclophosphamide, mycophenolate mofetil, and gusperimus. Examples of the anticancer agent include paclitaxel and docetaxel. Examples of the antibiotic include mitomycin and adriamycin. Examples of the antithrombotic drug include aspirin, ticlopidine, and argatroban. Examples of the HMG-CoA reductase inhibitor include cerivastatin. Examples of the ACE inhibitor include quinapril. Examples of the calcium antagonist include nifedipine. Examples of the antilipemic drug include probucol. Examples of the integrin inhibitor include AJM300. Examples of the antiallergic agent include tranilast. Examples of the antioxidant include α-tocopherol. Examples of the GPIIb/IIIa antagonist include abciximab. Examples of the retinoid include all-trans-retinoic acid. Examples of the lipid improver include eicosapentaenoic acid. Examples of the antiplatelet agent include clopidogrel. Examples of the anti-inflammatory agent include dexamethasone and prednisolone. Examples of the hemostatic agent include thrombin and collagen. Examples of the angiogenic inhibitor include sunitinib. Examples of the angiogenic agent include RGD protein. However, the present invention is not limited thereto.

The third polymer in the third coating layer 33 supports the drug and sustainably releases the drug. In general, in order to sustainably release the drug, it is preferable that the drug is dispersed in a non-porous polymer. Even if the drug is dispersed in the polymer, when the polymer is porous, the drug may be released early through the pores of the polymer. Therefore, the third polymer is preferably non-porous. Also, the release rate of the drug from the polymer supporting the drug generally depends on the diffusion rate of the drug in the polymer in the case of a non-biodegradable polymer, and depends on the degradation rate of the polymer in addition to the diffusion rate of the drug in the polymer in the case of a biodegradable polymer. The diffusion rate of the drug in the polymer correlates with the flexibility of the polymer. Therefore, it is known that the release rate of the drug can be controlled by adjusting the flexibility and degradation rate of the polymer. In addition, it is known that the polymer surface causes a foreign-body reaction to a living body, and such a foreign-body reaction contributes to an inflammatory reaction. A biodegradable polymer in which the polymer-derived inflammatory reaction disappears in the future is more preferable than a non-biodegradable polymer in which the polymer-derived inflammatory reaction may persist chronically. From the above, the third polymer is preferably a non-porous biodegradable polymer having moderate flexibility and degradation rate. The non-porous state of the polymer can be confirmed by a scanning electron microscope (SEM) or the like. In the present invention, the polymer being non-porous refers to a polymer having no pores that can be confirmed by a SEM. For example, a polymer in which a gap of about 0.1 µm can be confirmed by SEM observation is regarded as being porous.

Examples of the third polymer include polylactic acid, polycaprolactone, polyglycolic acid, polydioxanone, polybutyrolactone, polyvalerolactone, polyhydroxybutyric acid, polytrimethylene carbonate, and copolymers, derivatives, or mixtures thereof. These polymers are hydrophobic and non-porous biodegradable polymers. Among them, a polymer having a lactic acid monomer unit is preferable as the third polymer because the degradation product thereof is an in vivo metabolite and such a polymer has excellent biological safety and an excellent release rate of the drug. For example, PLLA, PDLA, PDLLA, poly(lactic-ε-caprolactone) which is a copolymer of lactic acid and ε-caprolactone, and poly(lactic-glycolic acid) which is a copolymer of lactic acid and glycolic acid are preferable. In particular, in the case of a copolymer of lactic acid and another monomer, the third polymer preferably has a lactic acid monomer unit because the flexibility is changed by adjusting the ratio between the lactic acid and another monomer, and the release rate can be adjusted. However, the third polymer is not limited thereto.

In an embodiment, the third polymer is poly(lactic-ε-caprolactone), which is a copolymer of lactic acid and ε-caprolactone. In this case, by containing caprolactone which is softer than other polymers as the third polymer, flexibility can be imparted to the third coating layer, so that peeling durability is improved.

In an embodiment of the present invention, the second polymer and the third polymer preferably have an identical monomer unit, and more preferably have a lactic acid monomer unit. With such a configuration, the second polymer and the third polymer have identical monomer unit, the affinity between the second polymer and the third polymer is improved, the formation of the interpenetrating polymer network structure between the second coating layer 32 and the third coating layer 33 is promoted, and the bonding force between the second coating layer 32 and the third coating layer 33 is increased, so that the peeling durability of the coating layer 30 is improved. In addition, when the third polymer has a lactic acid monomer unit, biological safety and sustained release of drug elution are improved. The content of the lactic acid monomer unit in the third polymer is preferably 50 mol% or more (upper limit: 100 mol%), and more preferably 70 mol% or more (upper limit: 100 mol%) with respect to all monomers constituting the third polymer.

The weight average molecular weight of the third polymer is preferably 100,000 to 1,000,000, and more preferably 150,000 to 800,000 from the viewpoint of peeling durability and the like.

Here, the third polymer favorably forms an interpenetrating polymer network structure with respect to the base polymer constituting the second polymer. The content of the third polymer is preferably the same as that of the base polymer constituting the second polymer, and the content ratio (weight ratio) between the third polymer and the base polymer is preferably 2 : 1 to 1 : 2, and more preferably 1.5 : 1 to 1 : 1.5.

As a preferred embodiment of the present invention, the third polymer has a lactic acid monomer unit, and the crosslinkable monomer is contained in an amount of 5 wt% or more and 90 wt% or less with respect to 100 wt% of the base polymer. As a more preferred embodiment of the present invention, the third polymer has a lactic acid monomer unit, and the crosslinkable monomer is contained in an amount of 30 wt% or more and 90 wt% or less with respect to 100 wt% of the base polymer. As a still more preferred embodiment of the present invention, the third polymer has a lactic acid monomer unit, and the crosslinkable monomer is contained in an amount of 30 wt% or more and 70 wt% or less with respect to 100 wt% of the base polymer.

The third coating layer 33 contains the drug and the third polymer at least in a part thereof, and may contain an additive other than the drug and the third polymer. Examples of the additive include elution aids that improve the diffusion rate of the drug in the polymer, such as dimethyl tartrate and diethyl tartrate, but the additive to be included in the third coating layer 33 is not limited thereto.

The primary structure, the secondary structure, and the higher order structure of the third polymer are not limited.

The drug in the third coating layer 33 is preferably uniformly dispersed, but is not limited thereto. In addition, the drug present in each of the dispersed regions may form a cluster in which a plurality of drug molecules is gathered. The third coating layer 33 may have nanoparticles, and the drug may also be present in a state of being encapsulated in the nanoparticles. The material of the nanoparticles is, for example, a copolymer of lactic acid and glycolic acid.

In the formation of the third coating layer 33, in order to prevent drug efficacy from being lost due to change of the original structure of the drug into an analogous substance, the third coating layer is formed without applying energy such as heat or light. Thus, the drug is supported by the third polymer having its original structure, and does not form a covalent bond with the third polymer. In addition, in the process of forming the third coating layer 33, a part of the drug may migrate into the second coating layer 32.

The thickness of the third coating layer 33 is not particularly limited, but is 0.1 to 200 µm, preferably 1 to 150 µm, more preferably 5 to 100 µm, and still more preferably 10 to 80 µm. The thickness of the third coating layer 33 may be uniform or non-uniform. The surface of the third coating layer 33 may be smooth or rough. The thickness of the third coating layer is measured by, for example, a laser microscope.

Here, a combination of the second polymer and the third polymer according to an embodiment of the present invention will be described. In an embodiment, when the base polymer of the second polymer has a lactic acid monomer unit, the crosslinkable monomer is a crosslinkable monomer having a di- or higher functional (preferably tetra- or higher functional) (meth)acrylate group; and the third polymer has a lactic acid monomer unit. In this case, a covalent bond between the first coating layer and the second coating layer and an interpenetrating polymer network structure between the second coating layer and the third coating layer are favorably formed, so that the desired effect of the present invention can be further exhibited.

For example, when the base polymer of the second polymer is polylactic acid, the crosslinkable monomer is preferably a crosslinkable monomer having a di- or higher functional (preferably tetra- or higher functional) (meth)acrylate group (for example, 1,4-butanediol diacrylate, pentaerythritol tetraacrylate, or dipentaerythritol hexaacrylate); and the third polymer is preferably polylactic acid or poly(lactic-ε-caprolactone).

For example, when the base polymer of the second polymer is poly(lactic-acid-ε-caprolactone), the crosslinkable monomer is a crosslinkable monomer having a di- or higher functional (preferably tetra- or higher functional) (meth)acrylate group (for example, 1,4-butanediol diacrylate, pentaerythritol tetraacrylate, or dipentaerythritol hexaacrylate); and the third polymer is poly-L-lactic acid or poly (lactic-ε-caprolactone).

In an embodiment, when the base polymer of the second polymer has a caprolactone unit, the crosslinkable monomer is a crosslinkable monomer having a di- or higher functional (preferably tetra- or higher functional) (meth)acrylate, an allyl group, or an acrylamide group; and the third polymer has a lactic acid monomer unit or a caprolactone unit. In this case, a covalent bond between the first coating layer and the second coating layer and an interpenetrating polymer network structure between the second coating layer and the third coating layer are favorably formed, so that the desired effect of the present invention can be exhibited.

For example, when the base polymer of the second polymer is polycaprolactone, the crosslinkable monomer is preferably a crosslinkable monomer having a di- or higher functional (preferably tetra- or higher functional) (meth)acrylate, an allyl group, or an acrylamide group; and the third polymer is poly-L-lactic acid or poly(lactic-ε-caprolactone).

### <Other coating layers>

Another coating layer may be further provided on the third coating layer 33. The other coating layer may have, for example, a function of suppressing the release rate of the drug in the third coating layer 33 to further impart a sustained release property, or a function of containing a drug different from the drug in the third coating layer 33 and assisting the drug efficacy of the drug in the third coating layer 33. The material of the other coating layer is not particularly limited.

### <Method for producing drug-eluting medical device 10>

Hereinafter, a method for producing the drug-eluting medical device 10 according to the present embodiment will be described.

A method for producing the drug-eluting medical device 10 according to the present embodiment includes: a first step of applying a first solution containing a dopamine molecule or an analog thereof as a first coating material (hereinafter, also referred to as "containing a first coating material") onto a substrate to form the first coating layer 31 in which the first coating material is polymerized; a second step of applying a second solution containing a polymer and/or a monomer as a second coating material (hereinafter, also referred to as "containing a second coating material") onto the first coating layer 31, and then performing heating or light irradiation to form the second coating layer 32; and a third step of applying a third solution containing a drug and a polymer as a third coating material (hereinafter, also referred to as "containing a third coating material") onto the second coating layer 32, and then performing drying to form the third coating layer 33. More specifically, the method for producing the drug-eluting medical device 10 according to the present embodiment includes: a first step of applying a first solution containing a dopamine molecule or an analog thereof as a first coating material onto a substrate and polymerizing the first coating material to form a first coating layer having a first polymer; a second step of applying a second solution containing a polymer and/or a monomer as a second coating material onto the first coating layer, and then performing heating or light irradiation to form a second coating layer having a second polymer; and a third step of applying a third solution containing a drug and a polymer as a third coating material onto the second coating layer, and then performing drying to form a third coating layer having a third polymer supporting the drug.

In the first step, a first solution containing a dopamine molecule or an analog thereof as a first coating material is applied to a substrate, and the first coating material is polymerized to form the first coating layer 31. Since the first coating layer 31 formed by such a step is polydopamine or a polymer similar to polydopamine, peeling durability between the substrate 20 and the first coating layer 31 is high, and additional coating with another material is facilitated.

The material of the substrate and the analog of the dopamine molecule are not particularly limited, but the materials exemplified in the description of the embodiment of the first coating layer 31 and the like can be applied. The solvent of the solution containing the first coating material is not particularly limited as long as the first coating material is soluble therein. In addition, the solution containing the first coating material may contain an additive such as a buffer. The first coating material may be in the form of a hydrate, a hydrochloride, or the like of a dopamine molecule or an analog thereof.

In the first step, the method for applying the first solution containing the first coating material to the substrate is not particularly limited. A preferred application method is a dip coating method. As the solvent of the first solution, for example, water, a buffer, a solvent used for a third solution to be described later, or the like is used. For example, when dopamine hydrochloride is used as the first coating material, water and a buffer are preferable as the solvent, and a tris-HCl buffer is more preferable. In the first solution, the content of the first coating material is preferably 0.01 to 30 mass%, more preferably 0.05 to 10 mass%, and still more preferably 0.01 to 5 mass% with respect to the total weight of the first solution. A dopamine molecule or an analog thereof is known to undergo autoxidation polymerization, and when a substrate is immersed in a solution containing them, the dopamine molecule or analog thereof undergoes autoxidation polymerization on the surface of the substrate to form a thin film on the substrate surface. The first coating layer 31 may also be formed by applying the first solution containing the first coating material to the substrate by another method, and then applying energy to perform polymerization.

In the first step, after a film in which the first coating material is polymerized is formed, steps such as a cleaning step, a drying step, and an annealing step may be added to form the first coating layer 31. With regard to the annealing step, peeling durability between the substrate and the first coating layer 31 can be improved by adding this step. The temperature and time for annealing are not particularly limited.

In the second step, the second coating layer 32 is formed by applying a second solution containing a polymer and/or a monomer as a second coating material onto the first coating layer 31, and then performing heating or light irradiation. The second coating material is any one of a combination of a base polymer and a crosslinkable monomer; a combination of a constituent monomer of the base polymer and a crosslinkable monomer; and a polymer having a functional group capable of covalently bonding to the first coating layer 31; or a combination thereof. As the base polymer and the crosslinkable monomer, the materials exemplified in the description of the embodiment of the second coating layer 32 can be applied. By applying the second solution containing the second coating material onto the first coating layer 31, and then performing heating or light irradiation, the second coating layer 32 in which the second coating material is crosslinked and/or polymerized is formed, a covalent bond is formed between the first coating layer 31 and the second coating layer 32, so that peeling durability between the first coating layer 31 and the second coating layer 32 is improved. That is, in an embodiment, in the second step, a second coating layer having a second polymer covalently bonded to the first polymer is formed by applying a second solution containing a polymer and/or a monomer as a second coating material onto the first coating layer, and then performing heating or light irradiation.

The solvent of the second solution containing the second coating material is not particularly limited as long as the second coating material is soluble therein. As the solvent of the second solution, for example, a solvent used for a third solution to be described later is similarly used. In addition, the solution containing the second coating material may contain additives such as a polymerization initiator and a buffer. The second coating material may also be in the form of a hydrate, a hydrochloride, or the like. In the second solution, the content ratio between the solvent and the second coating material is not particularly limited, but is preferably 10 to 800 mL, more preferably 20 to 600 mL, and still more preferably 100 to 500 mL with respect to 1 part by weight of the second coating material.

In the second step, the method for applying the second solution containing the second coating material is not particularly limited. In addition, steps such as a cleaning step, a drying step, and an annealing step may be added before heating or light irradiation or after heating or light irradiation. Preferably, a drying step is added before heating or light irradiation. When the drying step is added after heating or light irradiation, the second coating material is crosslinked and/or polymerized in a state of containing the solvent, so that the second coating layer 32 becomes porous, the mechanical strength of the second coating layer and the bonding force with the first coating layer 31 and/or the third coating layer 33 are lowered, and the peeling durability of the coating layer 30 may be lowered. Therefore, by adding the drying step before heating or light irradiation, the second coating layer 32 becomes non-porous, and the peeling durability of the coating layer 30 is improved. The drying conditions are not particularly limited.

In the second step, conditions such as the heating temperature, the heating time, the wavelength of irradiation light, and the integrated light amount at the time of heating or light irradiation are not particularly limited. In addition, if crosslinking and/or polymerization can be promoted, energy can be applied by a method other than heating or light irradiation.

In the third step, the third coating layer 33 is formed by applying a third solution containing a drug and a polymer as a third coating material onto the second coating layer 32, and then performing drying. As the drug and the polymer of the third coating material, the materials exemplified in the description of the embodiment of the third coating layer 33 can be applied. By applying the third solution containing the third coating material onto the second coating layer 32, and then performing drying, the third coating layer 33 in which the drug is supported on the polymer while having its original structure and is sustainably released is formed, and an interpenetrating polymer network is formed between the second coating layer 32 and the third coating layer 33, so that peeling durability between the second coating layer 32 and the third coating layer 33 is improved.

In the third step, a third solution containing a drug and a polymer as a third coating material is applied, and then drying is performed without heating or light irradiation. By avoiding heating or light irradiation, there is no risk that heat or light breaks a chemical bond in the drug, and an analogous substance in which the original structure of the drug has been changed is not generated. Therefore, the drug efficacy is not lost, and there is no risk that the analog substance causes side effects. However, those obtained by heating or light irradiation are not necessarily out of the technical scope of the present invention. Those obtained by heating or light irradiation are also included in the technical scope of the present invention as long as heating or light irradiation is performed at a weak level that does not cause a change in the original structure of the drug. That is, in the third step, after the third solution containing a drug and a polymer as a third coating material is applied, drying is performed without heating or light irradiation at a level that causes a change in the original structure of the drug.

As a preferred embodiment of the present invention, at least a part of the second coating material is soluble in the solvent of the third solution containing the third coating material, and in the third step, the second coating layer 32 swells when the third solution containing the third coating material is applied onto the second coating layer 32. With such a configuration, the polymer as the third coating material easily penetrates into the second coating layer 32, the formation of the interpenetrating polymer network structure between the second coating layer 32 and the third coating layer 33 is promoted, and the bonding force between the second coating layer 32 and the third coating layer 33 is increased, so that the peeling durability of the coating layer is improved.

The solvent of the third solution containing the third coating material is not particularly limited as long as the second coating material and the third coating material are soluble therein. Examples of the solvent include, but are not limited to, dimethylacetamide, dimethylformamide, tetrahydrofuran, cyclohexanone, acetone, acetonitrile, propylene glycol monomethyl ether, methyl butyl ketone, methyl ethyl ketone, diethyl ketone, ethyl acetate, n-butyl acetate, dioxane, chloroform, dimethyl sulfoxide, dimethylformamide, benzene, toluene, xylene, hexane, cyclohexane, pentane, heptane, octane, nonane, decane, decalin, isobutyl acetate, isopropyl acetate, diacetone alcohol, benzyl alcohol, 1-butanone, N-methylpyrrolidone, methylene chloride, carbon tetrachloride, tetrachloroethylene, tetrachloroethane, chlorobenzene, 1,1,1-trichloroethane, formamide, hexafluoroisopropanol (hexafluoro-2-propanol (HFIP)), 1,1,1-trifluoroethanol, hexamethylphosphoramide, and combinations thereof. From the viewpoint of compatibility between the second polymer and the third polymer, solvents such as tetrahydrofuran, acetone, acetonitrile, methyl butyl ketone, methyl ethyl ketone, diethyl ketone, chloroform, methylene chloride, carbon tetrachloride, tetrachloroethylene, tetrachloroethane, 1,1,1-trichloroethane, hexafluoroisopropanol, and 1,1,1-trifluoroethanol are preferable, and chloroform, methylene chloride, carbon tetrachloride, tetrachloroethylene, tetrachloroethane, 1,1,1-trichloroethane, hexafluoroisopropanol, and 1,1,1-trifluoroethanol are more preferable. In the third solution, the content ratio between the solvent and the third coating material is preferably 10 to 100 mL, more preferably 20 to 80 mL, and still more preferably 30 to 70 mL with respect to 1 part by weight of the third coating material.

In addition, the third solution containing the third coating material may contain an additive such as a buffer. The third coating material may also be in the form of a hydrate, a hydrochloride, or the like.

In the third step, the method for applying the third solution containing the third coating material is not particularly limited. In addition, the drying conditions at the time of drying after applying the third solution containing the third coating material are not particularly limited.

Although the embodiments of the present invention have been described in detail, this is illustrative and exemplary and not restrictive, and it is clear that the scope of the present invention should be interpreted by the appended claims.

Examples

Hereinafter, the peeling durability which is an effect of the drug-eluting medical device that achieves the object of the present invention will be described using the following Examples. However, the following Examples illustrate a part of the configuration of the drug-eluting medical device that achieves the object of the present invention. The configuration of the drug-eluting medical device that achieves the object of the present invention is not limited only to these Examples.

In the following Examples, since the third solution contains only the third polymer, the coating layer does not contain a drug. However, when the third polymer is non-porous, long-term sustained drug release is possible. Therefore, it can be understood by those skilled in the art that the sample obtained in this Example exhibits long-term sustained release.

### <Preparation of solution containing first coating material (first solution)>

Dopamine hydrochloride was mixed into 10 mM tris-HCl buffer (pH 8.5) to adjust the first solution. The concentration of dopamine hydrochloride in the first solution was 0.2 wt%.

### <Preparation of solution containing second coating material (second solution)>

A total of 74 types of second solutions described in Tables 3 to 6 were prepared by mixing, as a base polymer, 1 g of any one polymer of four types of poly-L-lactic acid (PLLA, BioDegmer (registered trademark) PLLA manufactured by BMG Inc., weight average molecular weight: 510,000), an L-lactic acid/ε-caprolactone copolymer having a molar ratio of L-lactic acid to ε-caprolactone of 75 : 25 (LCL7525, BMG Inc., BioDegmer (registered trademark) LCL (75 : 25), molecular weight: 570,000), poly-D,L-lactic acid (PDLLA, manufactured by DURECT Corporation, molecular weight: 102,000), and polycaprolactone (PCL, manufactured by Evonik Industries AG, intrinsic viscosity: 1.13 to 1.38 dL/g); as a crosslinkable monomer, any one crosslinking agent of five types of 1,4-butanediol diacrylate (1,4-BDDA), pentaerythritol tetraacrylate (PETA), dipentaerythritol hexaacrylate (DPEHA), triallyl isocyanate (TAIC), and N-[tris(3-acrylamidopropoxymethyl)methyl]acrylamide (FOM-03006) in any one of six weights of 0.05 g, 0.10 g, 0.30 g, 0.50 g, 0.70 g, and 0.90 g; 0.01 g of 2-hydroxy-1-[4-(2-hydroxyethoxy)phenyl]-2-methyl-1-propanone (IRGACURE2959); and 300 ml of chloroform.

### <Preparation of solution containing third coating material (third solution)>

A total of four types of third solutions were prepared by mixing 1 g of any one polymer of four types of PLLA, LCL7525, a DL-lactic acid/ε-caprolactone copolymer (DLCL9010) having a ratio of DL-lactic acid to ε-caprolactone of 90 : 10 (mol%), and polyvinyl alcohol (manufactured by FUJIFILM Wako Pure Chemical Corporation, product code 160-11485) (PVA) with 40 ml of a solvent. When PLLA, LCL7525, and DLCL9010 were used as a polymer, chloroform was used as a solvent, and the polymer was dissolved in chloroform at room temperature. When PVA was used as a polymer, water was used as a solvent, and the polymer was dissolved in water at 80°C. In the present invention, when PLLA, LCL7525, or DLCL9010 is used as a polymer in the third solution, the configuration of the resulting coating layer is the configuration of Examples, and when PVA is used as a polymer in the third solution, the configuration of the resulting coating layer is the configuration of Comparative Examples.

### <Preparation of first coating layer>

A sheet material of SUS304 (size: length 150 mm × width 70 mm × thickness 0.8 mm) was immersed in an aluminum tray filled with the first solution. After standing for 24 hours, the sample was taken out, and the surface thereof was rinsed with distilled water. Thereafter, the sample was dried at room temperature in the air for 2 hours. Then, the sample was allowed to stand on a hot plate heated at 200°C in the air for 5 minutes to perform an annealing treatment. The thickness of the obtained first coating layer was 40 nm as a result of measurement by an AFM (measuring instrument: Bruker Nano Surfaces, Dimension Icon). Hereinafter, the polymer formed in the first coating layer is referred to as a first polymer.

### <Preparation of second coating layer>

The sample on which the first coating layer has been formed was immersed in an aluminum tray filled with the second solution, and immediately taken out. Thereafter, the sample was dried at room temperature in the air for 1 hour. Then, the sample was dried in a vacuum oven at 40°C for 24 hours. The dried sample was placed in a polyethylene bag, and the inside of the bag was irradiated with ultraviolet rays having a wavelength of 365 nm from the outside of the bag so that an integrated light amount was 300 J/cm² in a state where the inside of the bag was purged with nitrogen. Thereafter, the sample was taken out from the bag, immersed in an aluminum tray filled with chloroform, and cleaned with an ultrasonic cleaner for 10 minutes to remove residual components such as unreacted monomers and a photopolymerization initiator. A second coating layer was prepared for each of the 74 types of the second solutions. The thickness of the obtained second coating layer was measured by an AFM (measuring instrument: Bruker Nano Surfaces, Dimension Icon). As a result, the thickness of a sample whose evaluation is described as "( )" in the following Tables 3 to 6 (a sample whose evaluation is surrounded by parentheses) was 50 nm to 100 nm, and the thickness of a sample whose evaluation is not surrounded by parentheses was 150 nm. Hereinafter, the polymer formed in the second coating layer is referred to as a second polymer.

### <Preparation of third coating layer>

About 0.02 ml of the third solution was dropped on the sample prepared up to the second coating layer with a Pasteur pipette. Thereafter, the sample was dried at room temperature in the air for 2 hours. Then, the sample was dried in a vacuum oven at 40°C for 72 hours. For each of all four types of the third solutions, a third coating layer was prepared on the surface of each of 74 types of samples prepared up to the second coating layer. The thickness of the obtained third coating layer was 30 µm as a result of measurement with a laser microscope (VK-X200, KEYENCE Corporation). The thickness of the third coating layer was calculated by comparing the thickness of the sample before the third coating layer was formed with the thickness of the sample after the third coating layer was formed. Hereinafter, the polymer formed in the third coating layer is referred to as a third polymer.

In the third polymer (PLLA, LCL7525, DLCL9010, PVA), the cross section of the sample was exposed by Ion Milling (IM4000Plus, Hitachi High-Tech Corporation), and then observed by a SEM (S-3400N, Hitachi High-Tech). As a result, it was observed that no observable pores were present, and it was confirmed that the third polymer was non-porous.

Here, in the case of using PLLA, LCL7525, and DLCL9010 as the third polymer, when the third solution was dropped on the second coating layer, the interference color of the coating film was visually observed on the dropped surface. This showed that the second coating layer was swollen by the third solution. On the other hand, in the case of using PVA as the third polymer, even when the third solution was dropped on the second coating layer, the interference color of the coating film was not observed on the dropped surface. In this case, it is considered that the second coating layer is not swollen.

As described above, samples of coating layers that coat the entire SUS sheet material, and include the first to third coating layers sequentially formed were obtained. In the following experiments, measurement is performed from one surface side of the coating layer.

### <Method of peeling durability test>

Samples of all 296 types of coating layers prepared up to the third coating layer were rubbed with brass tweezers from the surface side of the third coating layer in the air, and evaluation of peeling durability and identification of the position of the interface where peeling occurred were performed. In addition, each sample was immersed in water at 37°C, the unrubbed portion was rubbed in the same manner, and evaluation of peeling durability and identification of the position of the interface where peeling occurred were performed. The identification of the interface was performed based on the presence or absence of an interference color derived from the second coating layer on the peeling surface observed at a magnification of 50 using a stereoscopic microscope (SMZ645, Nikon Corporation). Specifically, as the "index of the position of the interface where peeling occurred", a case where no interference color derived from the second coating layer was observed on the peeling surface was defined as "A", a case where an interference color derived from the second coating layer was changed on the peeling surface or an interference color derived from the second coating layer was partially observed on the peeling surface was defined as "B", and a case where an interference color derived from the second coating layer was observed on the peeling surface in the same manner as in the formation of the second coating layer was defined as "C". Here, when the index of peeling durability is "1" or more, it can be determined that an interpenetrating polymer network structure is formed. When the index of the peeling durability is "0", it is considered that no interpenetrating polymer network structure is formed. That is, in the evaluations of the following Tables 3 to 6, the evaluation "0C" indicates that no interpenetrating polymer network structure is formed, and evaluations other than "OC" (for example, "1C" and the like) indicate that an interpenetrating polymer network structure is formed.

### <Result of peeling durability test>

The results of the peeling durability test of each sample are collectively shown in Tables 3 to 6 using the indices shown in Table 1 for the evaluation of peeling durability, and using the indices shown in Table 2 for the results of identifying the position of the interface where peeling occurred. In Tables 3 to 6, "( )" (evaluation in parentheses) represents a test result of a sample in which a part of the second coating layer disappeared when ultrasonic cleaning with chloroform was performed at the time of preparing the second coating layer, "Dry" represents a test result in the air, and "Wet" represents a test result in water.

**[Table 1]**

| Table 1 Index of peeling durability | | | |
|---|---|---|---|
| Index | Magnitude of force required for peeling | Presence or absence of loss of shape of coating layer at peeling | Region peeled by one time of rubbing in coating layer |
| 0 | Little | Absent | Entire |
| 1 | Very weak | Absent | Entire |
| 2 | Weak | Absent | Entire |
| 3 | Normal | Absent | Entire |
| 4 | Slightly strong | Present | Entire |
| 5 | Strong | Present | Part |
| 6 | Very strong | Present | Part |

**[Table 2]**

| Table 2 Index of position of interface at which peeling occurs | |
|---|---|
| Index | Position of interface at which peeling occurs |
| A | Between first coating layer and second coating layer |
| B | Inside second coating layer |
| C | Between second coating layer and third coating layer |

**[Table 3]**

| | | | Example | | | | | | Comparative Example | |
|---|---|---|---|---|---|---|---|---|---|---|
| Second polymer/base polymer | | | PLLA | | | | | | PLLA | |
| Third polymer | | | PLLA | | LCL7525 | | DLCL9010 | | PVA | |
| Evaluation environment | | | Dry | Wet | Dry | Wet | Dry | Wet | Dry | Wet |
| | Structure | Weight ratio^{*1} | | | | | | | | |
| | 1, 4-BDDA | 50 wt% | (3A) | (3A) | (5A) | (4A) | (5A) | (5A) | (0C) | (0C) |
| | | 70 wt% | (5A) | (5A) | (5A) | (5A) | (5A) | (5A) | (0C) | (0C) |
| | | 90 wt% | (5A) | (5A) | (4A) | (4A) | (4A) | (4A) | (0C) | (0C) |
| | PETA | 30 wt% | 5A | 5A | 5A | 5A | 6A | 5A | 0C | 0C |
| | | 50 wt% | 6A | 6A | 6A | 6A | 6A | 5A | 0C | 0C |
| Second polymer / crosslinkable monomer | | 70 wt% | 4B | 4B | 5B | 4B | 5B | 5B | 0C | 0C |
| | | 90 wt% | 4B | 4B | 5B | 4B | 6B | 5B | 0C | 0C |
| | DPEHA | 30 wt% | 6A | 6A | 6A | 6A | 6A | 6A | 0C | 0C |
| | | 50 wt% | 6B | 6B | 6B | 6B | 6B | 6B | 0C | 0C |
| | | 70 wt% | 6B | 6B | 5B | 5B | 6B | 6B | 0C | 0C |
| | | 90 wt% | 2C | 1C | 2C | 2C | 4C | 4C | 0C | 0C |
| | FOM-03006 | 30 wt% | (1C) | (1C) | (2C) | (1C) | (3C) | (3C) | (0C) | (DC) |
| | | 50 wt% | 1C | 1C | 2C | 1C | 4C | 2C | 0C | 0C |
| | | 70 wt% | 1C | 1C | 2C | 1C | 3C | 1C | 0C | 0C |
| | | 90 wt% | 1C | 1C | 1C | 1C | 2C | 1C | 0C | 0C |

| | | | | | | | | | | |
|---|---|---|---|---|---|---|---|---|---|---|
| *1: Weight ratio of crosslinkable monomer to base polymer PLLA (100 wt%) | | | | | | | | | | |

**[Table 4]**

| | | | Example | | | | | | Comparative Example | |
|---|---|---|---|---|---|---|---|---|---|---|
| Second polymer/base polymer | | | LCL7525 | | | | | | LCL7525 | |
| Third polymer | | | PLLA | | LCL7525 | | DLCL9010 | | PVA | |
| Evaluation environment | | | Dry | Wet | Dry | Wet | Dry | Wet | Dry | Wet |
| | Structure | Weight ratio^{*1} | | | | | | | | |
| | 1, 4-BDDA | 50 wt% | (2A) | (lA) | (3A) | (1A) | (4A) | (3A) | (0C) | (0C) |
| | | 70 wt% | (4A) | (3A) | (4A) | (3A) | (5A) | (3A) | (0C) | (0C) |
| | | 90 wt% | (4A) | (3A) | (4A) | (2A) | (5A) | (3A) | (0C) | (0C) |
| | PETA | 5 wt% | (1A) | (1A) | (2A) | (1A) | (4A) | (2A) | (0C) | (0C) |
| | | 10 wt% | 3A | 4A | 4A | 3A | 5A | 4A | 0C | 0C |
| | | 30 wt% | 4A | 5A | 5A | 4A | 5A | 4A | 0C | 0C |
| | | 50 wt% | 6A | 5A | 6A | 5A | 6A | 5A | 0C | 0C |
| Second polymer / crosslinkable monomer | | 70 wt% | 6A | 5A | 6A | 5A | 6A | 6A | 0C | 0C |
| | | 90 wt% | 4B | 4B | 5B | 3B | 5B | 4B | 0C | 0C |
| | DPEHA | 5 wt% | 4A | 5A | 5A | 4A | 5A | 5A | 0C | 0C |
| | | 10 wt% | 6A | 5A | 5A | 5A | 6A | 5A | 0C | 0C |
| | | 30 wt% | 6A | 4A | 6A | 5A | 6A | 5A | 0C | 0C |
| | | 50 wt% | 6B | 5B | 6B | 5B | 6A | 5A | 0C | 0C |
| | | 70 wt% | 5B | 3B | 5B | 4B | 5B | 4B | 0C | 0C |
| | | 90 wt% | 4C | 3C | 4B | 3B | 5B | 4B | 0C | 0C |
| | FOM-03006 | 30 wt% | 1C | 1C | 2C | 2C | 3C | 2C | 0C | 0C |
| | | 50 wt% | 1C | 1C | 2C | 1C | 2C | 2C | 0C | 0C |
| | | 70 wt% | 1C | 1C | 1C | 1C | 2C | 1C | 0C | 0C |
| | | 90 wt% | 1C | 1C | 1C | 1C | 2C | 3C | 0C | 0C |

| | | | | | | | | | | |
|---|---|---|---|---|---|---|---|---|---|---|
| *1: Weight ratio of crosslinkable monomer to base polymer LCL7525 (100 wt%) | | | | | | | | | | |

**[Table 5]**

| | | | Example | | | | | | Comparative Example | |
|---|---|---|---|---|---|---|---|---|---|---|
| Second polymer/base polymer | | | PDLLA | | | | | | PDLLA | |
| Third polymer | | | PLLA | | LCL7525 | | DLCL9010 | | PVA | |
| Evaluation environment | | | Dry | Wet | Dry | Wet | Dry | Wet | Dry | Wet |
| | Structure | Weight ratio^{*1} | | | | | | | | |
| | 1, 4-BDDA | 70 wt% | (3A) | (3A) | (4A) | (3A) | (4A) | (5A) | (0C) | (0C) |
| | | 90 wt% | (3A) | (3A) | (4A) | (4A) | (5A) | (4A) | (0C) | (0C) |
| | PETA | 30 wt% | 5A | 6A | 4B | 6B | 6A | 5A | 0C | 0C |
| | | 50 wt% | 5B | 6B | 5B | 6B | 6A | 6A | 0C | 0C |
| | | 70 wt% | 6B | 6B | 5B | 6B | 6A | 6A | 0C | 0C |
| Second polymer / crosslinkable monomer | | 90 wt% | 6B | 6B | 6B | 5B | 6B | 5B | 0C | 0C |
| | DPEHA | 30 wt% | 6A | 6A | 6A | 6A | 6A | 5A | 0C | 0C |
| | | 50 wt% | 6B | 6B | 6B | 6B | 6B | 5B | 0C | 0C |
| | | 70 wt% | 6B | 6B | 5B | 5B | 6B | 6B | 0C | 0C |
| | | 90 wt% | 3C | 3C | 2C | 3C | 4C | 5B | 0C | 0C |
| | FOM-03006 | 30 wt% | (1C) | (1C) | (2C) | (1C) | (4C) | (3C) | (0C) | (0C) |
| | | 50 wt% | 1C | 1C | 1C | 2C | 4C | 3C | 0C | 0C |
| | | 70 wt% | 1C | 1C | 2C | 1C | 4C | 3C | 0C | 0C |
| | | 90 wt% | 1C | 1C | 1C | 2C | 4C | 3C | 0C | 0C |

| | | | | | | | | | | |
|---|---|---|---|---|---|---|---|---|---|---|
| *1: Weight ratio of crosslinkable monomer to base polymer PDLLA (100 wt%) | | | | | | | | | | |

**[Table 6]**

| | | | Example | | | | | | Comparative Example | |
|---|---|---|---|---|---|---|---|---|---|---|
| Second polymer/base polymer | | | PCL | | | | | | PCL | |
| Third polymer | | | PLLA | | LCL7525 | | DLCL9010 | | PVA | |
| Evaluation environment | | | Dry | Wet | Dry | Wet | Dry | Wet | Dry | Wet |
| | Structure | Weight ratio^{*1} | | | | | | | | |
| | 1, 4-BDDA | 70 wt% | (1A) | (1A) | (1A) | (1A) | (2A) | (2A) | (0C) | (0C) |
| | | 90 wt% | (1A) | (1A) | (1A) | (2A) | (2A) | (2A) | (0C) | (0C) |
| | PETA | 5 wt% | (1C) | (1C) | (2A) | (2A) | (2A) | (2A) | (0C) | (0C) |
| | | 10 wt% | (1C) | (1C) | (1A) | (3A) | (2A) | (3A) | (0C) | (0C) |
| | | 30 wt% | (1C) | (1C) | (1A) | (2A) | (2A) | (3A) | (0C) | (0C) |
| | | 50 wt% | (1C) | (1C) | (2B) | (2B) | (2C) | (3C) | (0C) | (0C) |
| | | 70 wt% | 1C | 1C | 2B | 2B | 2C | 3C | 0C | 0C |
| | | 90wt% | 1C | 1C | 2C | 2C | 3C | 3C | 0C | 0C |
| | DPEHA | 5 wt% | (1C) | (1C) | (2A) | (2A) | (2A) | (3A) | (0C) | (0C) |
| | | 10 wt% | (1C) | (1C) | (2A) | (1A) | (2A) | (3A) | (0C) | (0C) |
| Second polymer / crosslinkable monomer | | 30 wt% | (1C) | (1C) | (3B) | (3B) | (3C) | (3C) | (0C) | (0C) |
| | | 50 wt% | 1C | 1C | 2C | 3C | 2C | 3C | 0C | 0C |
| | | 70 wt% | 1C | 1C | 2C | 3C | 2C | 3C | 0C | 0C |
| | | 90 wt% | 1C | 1C | 2C | 3C | 2C | 4C | 0C | 0C |
| | TAIC | 5 wt% | (1A) | (1A) | (1A) | (1A) | (2A) | (2A) | (0C) | (DC) |
| | | 10 wt% | (1C) | (1C) | (1A) | (1A) | (2A) | (3A) | (0C) | (0C) |
| | | 30 wt% | 1C | 1C | 1A | 1A | 2A | 2A | 0C | 0C |
| | | 50 wt% | 1C | 1C | 1A | 1A | 2A | 2A | 0C | 0C |
| | | 70 wt% | 1C | 1C | 1A | 1A | 2A | 2A | 0C | 0C |
| | | 90 wt% | 1C | 1C | 1A | 1A | 2A | 2A | 0C | 0C |
| | FOM-03006 | 5 wt% | (1A) | (1A) | (2A) | (2A) | (3A) | (2A) | (0C) | (0C) |
| | | 10 wt% | (1C) | (1C) | (2A) | (2A) | (3A) | (2A) | (0C) | (0C) |
| | | 30 wt% | 1C | 1C | 2B | 2B | 3A | 3C | 0C | 0C |
| | | 50 wt% | 1C | 1C | 3B | 2B | 3A | 3C | 0C | 0C |
| | | 70 wt% | 1C | 1C | 2C | 2C | 3C | 3C | 0C | 0C |
| | | 90 wt% | 1C | 1C | 2C | 1C | 3C | 2C | 0C | 0C |

| | | | | | | | | | | |
|---|---|---|---|---|---|---|---|---|---|---|
| *1: Weight ratio of crosslinkable monomer to base polymer PCL (100 wt%) | | | | | | | | | | |

As shown in Tables 3 to 6, it was shown that coating layers formed from any of PLLA, LCL7525, PDLLA, and PCL as a base polymer of the second polymer; any of 1,4-BDDA, PETA, DPEHA, TAIC, and FOM-03006 as a crosslinkable monomer of the second polymer; any of PLLA, LCL7525, and DLCL9010 as the third polymer had good peeling durability. On the other hand, as shown in Tables 3 to 6, it was found that coating layers formed from any of PLLA, LCL7525, PDLLA, and PCL as a base polymer of the second polymer; any of 1,4-BDDA, PETA, DPEHA, TAIC, and FOM-03006 as a crosslinkable monomer of the second polymer; and PVA as the third polymer had insufficient peeling durability.

That is, when PVA was used as the third polymer, the index of the peeling durability was "0", indicating that the peeling durability was low. When considered together with the index of the position of the interface where peeling occurred, the result of peeling durability was "0C", and this suggests that when PVA was used as the third polymer, an interpenetrating polymer network structure with the second polymer was not formed. This is presumed to be because the solvent of the third solution when the third polymer is PVA is water, and thus even when the third solution was dropped on the second coating layer having the hydrophobic second polymer, the second coating layer did not swell. Actually, at the time of forming the third coating layer, the interference color of the coating film when the third solution was dropped on the second coating layer was not observed, and this suggested that the second coating layer did not swell. The above results showed that PVA is not suitable as the third polymer in the present invention.

In the coating layers of Examples, when the crosslinkable monomer was 1,4-BDDA, PETA, or DPEHA, peeling durability was generally higher than that of TAIC or FOM-03006. Thus, in the embodiment of the present invention, the crosslinkable monomer is more preferably an acrylate. As a cause of this, the following is considered: since the reactivity of the acryloyl group which is a functional group of the acrylate is good, the covalent bond between the first coating layer and the second coating layer is sufficient; and the compatibility between the acrylate and the base polymer used in this time is good, a sufficient crosslinked structure is formed between the crosslinkable monomer and the base polymer, and the polymer component is retained even during ultrasonic cleaning with chloroform, so that a sufficient interpenetrating polymer network structure could be formed between the third coating layer. When the crosslinkable monomer is methacrylate, since the reactivity of the methacryloyl group which is a functional group is equivalent to that of the acryloyl group, the peeling durability is presumed to be similarly high. Thus, in the embodiment of the present invention, the crosslinkable monomer is more preferably a (meth)acrylate.

In the coating layers of Examples, when the crosslinkable monomer was PETA or DPEHA, a tendency was obtained that a large force was required for peeling as compared with the case where the crosslinkable monomer was 1,4-BDDA. This is considered to be because the number of covalent bonds formed between the first coating layer and the second coating layer is larger in the case of tetra- or higher functionality than in the case of less than tetra functionality, and therefore the bonding force between the first coating layer and the second coating layer was high.

In the coating layers of Examples, when the base polymer had a lactic acid monomer unit, peeling durability was generally higher than that when the base polymer was PCL. Therefore, in the embodiment of the present invention, the third polymer and the base polymer more preferably have a lactic acid monomer unit. As a factor of this, the following is considered: since the third polymer in the third coating layer has a lactic acid monomer unit, the base polymer having a lactic acid monomer unit has good affinity with the third polymer than the base polymer having no lactic acid monomer unit, so that the formation of an interpenetrating polymer network structure between the second coating layer and the third coating layer was promoted. Even in the case of using PCL as the base polymer, when the third polymer was LCL7525 or DLCL9010, peeling durability was generally higher than that when the third polymer was PLLA. This is considered to be because the presence of the caprolactone monomer unit in both the base polymer and the third polymer improved the affinity of both polymers, and promoted the formation of an interpenetrating polymer network structure between the second coating layer and the third coating layer. From the above, it is suggested that, when not only the lactic acid monomer unit and/or the caprolactone monomer unit are contained, but also the second polymer and the third polymer have an identical monomer unit, the affinity between the second polymer and the third polymer are improved, the formation of an interpenetrating polymer network structure between the second coating layer and the third coating layer is promoted, and the bonding force between the second coating layer and the third coating layer is increased, so that the peeling durability of the coating layer is improved.

In the coating layers of Examples, when the crosslinkable monomer was PETA or DPEHA, the base polymer had a lactic acid monomer unit, and the crosslinkable monomer was contained in an amount of 5 wt% or more and 90 wt% or less with respect to the weight (100 wt%) of the base polymer, peeling durability was generally high. Therefore, in the embodiment of the present invention, it is more preferable that the third polymer and the base polymer have a lactic acid monomer unit, the crosslinkable monomer is a tetra- or higher functional acrylate, and the crosslinkable monomer is contained in an amount of 5 wt% or more and 90 wt% or less with respect to the weight (100 wt%) of the base polymer. It is suggested that by adopting such a configuration, a covalent bond between the first coating layer and the second coating layer is sufficiently formed, and the formation of an interpenetrating polymer network between the second coating layer and the third coating layer is promoted.

In the coating layers of Examples, when the crosslinkable monomer was PETA or DPEHA, the base polymer had a lactic acid monomer unit, and the crosslinkable monomer was contained in an amount of 30 wt% or more and 90 wt% or less with respect to the weight (100 wt%) of the base polymer, peeling durability was generally high. Therefore, in the embodiment of the present invention, it is more preferable that the third polymer and the base polymer have a lactic acid monomer unit, the crosslinkable monomer is a tetra- or higher functional acrylate, and the crosslinkable monomer is contained in an amount of 30 wt% or more and 90 wt% or less with respect to the weight (100 wt%) of the base polymer. It is suggested that by adopting such a configuration, a covalent bond between the first coating layer and the second coating layer is sufficiently formed, and the formation of an interpenetrating polymer network structure between the second coating layer and the third coating layer is promoted. Even when the third polymer and the base polymer have a lactic acid monomer unit, the crosslinkable monomer is a tetrafunctional acrylate, the base polymer further has a caprolactone monomer unit, and the crosslinkable monomer is contained in an amount of 10 wt% or more and 30 wt% or less with respect to the weight (100 wt%) of the base polymer, and even when the third polymer and the base polymer have a lactic acid monomer unit, the crosslinkable monomer is a penta- or higher functional acrylate, the base polymer further has a caprolactone monomer unit, and the crosslinkable monomer is contained in an amount of 5 wt% or more and 30 wt% or less with respect to the weight (100 wt%) of the base polymer, peeling durability is high, which is preferable in the embodiment of the present invention.

In the coating layers of Examples, when the crosslinkable monomer was PETA or DPEHA, the base polymer had a lactic acid monomer unit, and the crosslinkable monomer was contained in an amount of 30 wt% or more and 70 wt% or less with respect to the weight (100 wt%) of the base polymer, peeling durability was generally high. Therefore, in the embodiment of the present invention, it is more preferable that the third polymer and the base polymer have a lactic acid monomer unit, the crosslinkable monomer is a tetra- or higher functional acrylate, and the crosslinkable monomer is contained in an amount of 30 wt% or more and 70 wt% or less with respect to the weight (100 wt%) of the base polymer. It is suggested that by adopting such a configuration, a covalent bond between the first coating layer and the second coating layer is sufficiently formed, and the formation of an interpenetrating polymer network structure between the second coating layer and the third coating layer is promoted. When the crosslinkable monomer was PETA or DPEHA, the base polymer had a lactic acid monomer unit, and the crosslinkable monomer was contained in an amount of 90 wt% with respect to the weight (100 wt%) of the base polymer, the position of the interface where peeling occurred was between the second coating layer and the third coating layer, and peeling durability was generally lower than that of the case where the crosslinkable monomer was contained in an amount of 30 wt% or more and 70 wt% or less with respect to the weight (100 wt%) of the base polymer. This is considered to be because the swelling property of the second coating layer when the third solution was applied onto the second coating layer was deteriorated due to increase in the proportion of the crosslinkable monomer in the second coating layer, and the formation of an interpenetrating polymer network structure between the second coating layer and the third coating layer was not relatively promoted. In fact, it has been confirmed that when the crosslinkable monomer was DPEHA, and the crosslinkable monomer was contained in an amount of 90 wt% with respect to the weight (100 wt%) of the base polymer, the swelling property of the second coating layer when the third solution was applied onto the second coating layer was lower than that of the case where the crosslinkable monomer was contained in an amount of 70 wt% or less. The reason why the position of the interface where peeling occurred was inside the second coating layer and the peeling durability was generally lower than that of the case where the crosslinkable monomer was contained in an amount of 30 wt% or more and 70 wt% or less with respect to the weight (100 wt%) of the base polymer is considered that the second coating layer became brittle due to increase in the proportion of the crosslinkable monomer in the second coating layer. In addition, since the position of the interface where peeling occurred was not between the first coating layer and the second coating layer, it is considered that the covalent bond between the first coating layer and the second coating layer was sufficiently formed. When the third polymer and the base polymer have a lactic acid monomer unit, the crosslinkable monomer is a tetrafunctional acrylate, and the crosslinkable monomer is contained in an amount of 70 wt% or more and 90 wt% or less with respect to the weight (100 wt%) of the base polymer, peeling durability is generally higher than that of the case where the third polymer and the base polymer have a lactic acid monomer unit, the crosslinkable monomer is a penta- or higher functional acrylate, and the crosslinkable monomer is contained in an amount of 70 wt% or more and 90 wt% or less with respect to the weight (100 wt%) of the base polymer, which is preferable in the embodiment of the present invention.

### <TEM observation of interpenetrating polymer network structure>

Next, an interface between the second coating layer and the third coating layer was observed using a scanning electron microscope (TEM) to confirm whether or not an interpenetrating polymer network structure was formed. Samples 1 and 2 of the coating layer having the configuration of Examples, and Sample 3 of the coating layer having the configuration of Comparative Examples were prepared according to the following method.

### [Preparation of Samples 1 to 3]

### <Preparation of solution containing second coating material (second solution)>

A second solution was prepared by mixing, as a base polymer, 1 g of a L-lactic acid/ε-caprolactone copolymer having a molar ratio of L-lactic acid to ε-caprolactone of 75 : 25 (LCL7525, BMG Inc., BioDegmer (registered trademark) LCL (75 : 25), molecular weight: 570,000); as a crosslinkable monomer, 0.05 g of dipentaerythritol hexaacrylate (DPEHA); 0.01 g of 2-hydroxy-1-[4-(2-hydroxyethoxy)phenyl]-2-methyl-1-propanone (IRGACURE2959); and 20 ml of chloroform.

### <Preparation of solution containing third coating material (third solution)>

A third solution was prepared by mixing 1 g of a DL-lactic acid/ε-caprolactone copolymer (DLCL9010) having a ratio of DL-lactic acid to ε-caprolactone of 90 : 10 (mol%) or polyvinyl alcohol (manufactured by FUJIFILM Wako Pure Chemical Corporation, product code 160-11485) (PVA), and 80 ml of a solvent. In Sample 1, DLCL9010 was used as the polymer, hexafluoro-2-propanol (HFIP) was used as the solvent, and the polymer was dissolved in HFIP at room temperature. In Sample 2, DLCL9010 was used as the polymer, chloroform was used as the solvent, and the polymer was dissolved in chloroform at room temperature. In Sample 3, PVA was used as the polymer, water was used as the solvent, and the polymer was dissolved in water at 80°C.

### <Preparation of film sheet made of second coating material>

The second solution was poured into a PFA petri dish of φ 75 mm so that air bubbles were not mixed, and air-dried at room temperature overnight to obtain a cast film. Next, the obtained film was placed in a polyethylene bag, and a film formed by irradiation with ultraviolet rays having a wavelength of 365 nm from the outside of the bag so that the integrated light amount of was 3,000 mJ/cm² in a state where the inside of the bag was purged with nitrogen was peeled off from the petri dish, to obtain a film sheet made of a second coating material.

### <Preparation of third coating layer>

About 0.02 ml of the third solution was dropped on the film sheet made of the second coating material with a Pasteur pipette. Thereafter, drying was performed at room temperature in the air for 2 hours. Then, drying was performed in a vacuum oven at 40°C for 72 hours.

In Samples 1 to 3, the thickness of the film sheet made of the second coating material was 200 to 300 µm as a result of measurement with a thickness gauge (model: 547-360, manufactured by Mitutoyo Corporation), and the thickness of the third coating layer was 3 to 6 µm as a result of measurement with a laser microscope (VK-X200, KEYENCE Corporation). The composition of each coating layer of the obtained Samples 1 to 3 was shown in Table 7 below.

**[Table 7]**

| | Second coating layer | | | Third coating layer | |
|---|---|---|---|---|---|
| | Base polymer | Crosslinkable monomer | Crosslinkable monomer weight ratio^{*1} | Third polymer | Solvent species of third solution |
| Sample 1 | LCL7525 | DPEHA | 5 wt% | DLCL9010 | HFIP |
| Sample 2 | LCL7525 | DPEHA | 5 wt% | DLCL9010 | CHCl₃ |
| Sample 3 | LCL7525 | DPEHA | 5 wt% | PVA | Water |

| | | | | | |
|---|---|---|---|---|---|
| *1: Weight ratio of crosslinkable monomer to base polymer PLLA (100 wt%) | | | | | |

Samples 1 to 3 having a coating layer of the composition in Table 7 were embedded in a resin. In Samples 1 and 2 as Examples, an epoxy resin (Epon812) was used as an embedding resin, and in Sample 3 as Comparative Examples, caprolactone (EVONIK C212) was used as an embedding resin. The hydrophobic polymer caprolactone was selected as the embedding resin of Sample 3 in order to avoid dissolution of PVA used as the third polymer in the epoxy resin (Epon812).

The sample embedded in a resin was sliced using an ultramicrotome (Leica EM UC7), and an enlarged image of the obtained thin section was photographed using a transmission electron microscope (model H-7100, manufactured by Hitachi, Ltd., acceleration voltage: 100 kV). The obtained TEM images (no staining, 25,000 times) are shown in Figs. 2 to 4. Fig. 2 is a TEM image of Sample 1, Fig. 3 is a TEM image of Sample 2, and Fig. 4 is a TEM image of Sample 3.

As shown in Figs. 2 and 3, in Samples 1 and 2, a region with non-uniform lightness was observed at the interface between the second coating layer and the third coating layer. On the other hand, as shown in Fig. 4, in Sample 3, the interface between the second coating layer and the third coating layer was clearly confirmed, and a region with non-uniform lightness was not observed between the second coating layer and the third coating layer. Since the lightness of the TEM image of the polymer material reflects the density, the average atomic number, and the like, it is suggested that the structure of the polymer is more uneven in the region where the lightness is uneven at the interface between the second coating layer and the third coating layer, confirmed in Samples 1 and 2, than in the regions of the second and third coating layers. Therefore, it can be determined that an interpenetrating polymer network structure is formed by the second polymer and the third polymer at the interface between the second coating layer and the third coating layer, and a non-uniform polymer structure is formed. "IPN" in Figs. 2 and 3 indicates a region where an interpenetrating polymer network structure is considered to be formed.

Here, the results of the peeling durability test of Samples 1 and 2 are shown in Table 8 below. The method of the peeling durability test is the same as the method described above. Sample 2 is the same as the system described in Table 4, in which LCL7525 as the base polymer and DPEHA as the crosslinkable monomer were used as the second polymer, and DLCL9010 (solvent species of the third solution: CHCl₃) was used as the third polymer.

**[Table 8]**

| | Example | | | |
|---|---|---|---|---|
| | Sample 1 | | Sample 2 | |
| Second polymer/base polymer | LCL7525 | | | |
| Second polymer/crosslinkable monomer | DPEHA | | | |
| Crosslinkable monomer weight ratio^{*1} | 5 wt% | | | |
| Third polymer | DLCL9010 | | | |
| Solvent species of third solution | HFIP | | CHCl₃ | |
| Evaluation environment | Dry | Wet | Dry | Wet |
| Peeling durability test | 5A | 5A | 5A | 5A |

| | | | | |
|---|---|---|---|---|
| *1: Weight ratio of crosslinkable monomer to base polymer LCL7525 (100 wt%) | | | | |

As shown in Table 8, the results of the peeling durability test of Samples 1 and 2 were the same. Therefore, in the third solution for preparing the third coating layer, it was confirmed that a similar interpenetrating polymer network structure was formed between the second coating layer and the third coating layer regardless of whether CHCl₃ or HFIP solvent was used as the solvent. Therefore, it was confirmed that a similar interpenetrating polymer network structure was formed between the second coating layer and the third coating layer even when CHCl₃ and HFIP were used as the solvent of the third solution for preparing the third coating layer.

### <Solution to problem and advantageous effects of invention>

Based on the above, solution to problem and advantageous effects of the invention will be described again below.

A drug-eluting medical device that achieves an object of the present invention includes: a substrate; a first coating layer having a first polymer obtained by autoxidation polymerization of a dopamine molecule or an analog thereof on the substrate; a second coating layer having a second polymer covalently bonded to the first polymer on the first coating layer; and a third coating layer having a drug and a third polymer supporting the drug on the second coating layer, wherein the second polymer and the third polymer form an interpenetrating polymer network structure.

With such a configuration, an effect is provided that the drug has an intended drug efficacy and drug elution is sustained release because the drug is supported by the third polymer while having its original structure, and the coating layer has high peeling durability because the third polymer forms an interpenetrating polymer network structure with the second polymer.

The second polymer and the third polymer may have an identical monomer unit.

With such a configuration, the affinity between the second polymer and the third polymer is improved, an interpenetrating polymer network structure between the second coating layer and the third coating layer is easily formed, and the bonding force between the second coating layer and the third coating layer is increased, so that the peeling durability of the coating layer is improved.

The second polymer may be a crosslinked polymer obtained by crosslinking a base polymer and a crosslinkable monomer.

With such a configuration, a covalent bond is formed between the functional group of the crosslinkable monomer and the first polymer, and the bonding force between the first coating layer and the second coating layer is improved, so that the peeling durability of the coating layer is improved. In addition, the functional group of the base polymer is not necessarily covalently bonded to the first polymer, and a wide variety of polymers can be selected as the base polymer.

The third polymer may have a lactic acid monomer unit.

With such a configuration, biological safety and sustained release of drug elution are improved.

The second polymer and the third polymer may have a lactic acid monomer unit.

With such a configuration, the affinity between the second polymer and the third polymer is improved, an interpenetrating polymer network structure between the second coating layer and the third coating layer is easily formed, and the bonding force between the second coating layer and the third coating layer is increased, so that the peeling durability of the coating layer is improved. In addition, biological safety and sustained release of drug elution are improved.

The base polymer may have a lactic acid monomer unit, and the crosslinkable monomer may be a (meth)acrylate.

With such a configuration, a covalent bond between the first coating layer and the second coating layer, and a crosslinked structure of the base polymer and the crosslinkable monomer are easily formed, so that peeling durability of the coating layer is improved.

The (meth)acrylate may be a tetra- or higher functional (meth)acrylate.

With such a configuration, a covalent bond is easily formed between the first coating layer and the second coating layer, so that the peeling durability of the coating layer is improved.

The third polymer and the base polymer may have a lactic acid monomer unit, the crosslinkable monomer may be a tetra- or higher functional acrylate, and the crosslinkable monomer may be contained in an amount of 5 wt% or more and 90 wt% or less with respect to the weight (100 wt%) of the base polymer.

With such a configuration, a covalent bond between the first coating layer and the second coating layer, and an interpenetrating polymer network structure between the second coating layer and the third coating layer are easily formed, so that the peeling durability of the coating layer is improved.

The third polymer and the base polymer may have a lactic acid monomer unit, the crosslinkable monomer may be a tetra- or higher functional acrylate, and the crosslinkable monomer may be contained in an amount of 30 wt% or more and 90 wt% or less with respect to the weight (100 wt%) of the base polymer.

With such a configuration, a covalent bond between the first coating layer and the second coating layer is more easily formed than the case where the crosslinkable monomer is contained in an amount of 5 wt% or more and less than 30 wt% with respect to the weight (100 wt%) of the base polymer, so that the peeling durability of the coating layer is improved.

The third polymer and the base polymer may have a lactic acid monomer unit, the crosslinkable monomer may be a tetra- or higher functional acrylate, and the crosslinkable monomer may be contained in an amount of 30 wt% or more and 70 wt% or less with respect to the weight (100 wt%) of the base polymer.

With such a configuration, an interpenetrating polymer network structure between the second coating layer and the third coating layer is more easily formed than the case where the crosslinkable monomer is contained in an amount of more than 70 wt% and 90 wt% or less with respect to the weight (100 wt%) of the base polymer, so that the peeling durability of the coating layer is improved.

In addition, the drug-eluting stent is particularly suitable as the drug-eluting medical device of the present invention because a force to peel the coating layer is likely to be applied at the time of mounting, delivery, and expansion, and elution of a drug that suppresses a biological reaction causing restenosis needs to last for at least several months or more. Therefore, the drug-eluting medical device may be a drug-eluting stent.

A method for producing a drug-eluting medical device that achieves an object of the present invention includes: a first step of applying a first solution containing a dopamine molecule or an analog thereof as a first coating material onto a substrate and polymerizing the first coating material to form a first coating layer having a first polymer; a second step of applying a second solution containing a polymer and/or a monomer as a second coating material onto the first coating layer, and then performing heating or light irradiation to form a second coating layer having a second polymer; and a third step of applying a third solution containing a drug and a polymer as a third coating material onto the second coating layer, and then performing drying to form a third coating layer having a third polymer supporting the drug.

By producing in such a step, a first coating layer composed of polydopamine or a polymer similar to polydopamine is formed in the first step. In the second step, a second coating layer in which the second coating material is crosslinked and/or polymerized is formed, and a covalent bond is formed between the first coating layer and the second coating layer. In addition, in the third step, a third coating layer in which the drug is supported on the polymer and is sustainably released is formed, an interpenetrating polymer network structure is formed between the second coating layer and the third coating layer, and the drug as the third coating material is supported on the polymer without losing its original drug efficacy. Therefore, it is possible to produce a drug-eluting medical device in which drug elution is sustained release and the peeling durability of the coating layer is high without losing the original drug efficacy of the drug.

At least a part of the second coating material may be soluble in a solvent of the third solution, and in the third step, the second coating layer may swell when the third solution is applied onto the second coating layer.

With such characteristics, the polymer as the third coating material easily penetrates into the second coating layer, the formation of the interpenetrating polymer network structure between the second coating layer and the third coating layer is promoted, the bonding force between the second coating layer and the third coating layer is increased, so that the peeling durability of the coating layer is improved.

The polymer constituting the second coating layer and the polymer constituting the third coating layer formed by the production method described above may have characteristics of the second polymer and the third polymer in the drug-eluting medical device that achieves an object of the present invention.

Specifically, in the method for producing a drug-eluting medical device, (1) the second polymer and the third polymer may have an identical monomer unit; (2) the third polymer may have a lactic acid monomer unit; (3) the second polymer and the third polymer may have a lactic acid monomer unit; (4) the second polymer may be a crosslinked polymer obtained by crosslinking a base polymer and a crosslinkable monomer; (5) in the above (4), the base polymer may have a lactic acid monomer unit, and the crosslinkable monomer may be a (meth)acrylate; (6) in the above (5), the (meth)acrylate may be a tetra- or higher functional (meth)acrylate; and/or (7) in the above (4) to (6), the third polymer may have a lactic acid monomer unit, and the crosslinkable monomer may be contained in an amount of 5 wt% or more and 90 wt% or less with respect to 100 wt% of the base polymer. Since the second polymer and the third polymer have the above characteristics, the peeling durability of the coating layer is improved.

The above-described method for producing a drug-eluting medical device may be a method for producing a drug-eluting stent.

### Reference Signs List

- 10: Drug-eluting medical device
- 20: Substrate
- 30: Coating layer
- 31: First coating layer
- 32: Second coating layer
- 33: Third coating layer

## Claims

1. A drug-eluting medical device comprising:
a substrate;
a first coating layer having a first polymer obtained by autoxidation polymerization of a dopamine molecule or an analog thereof on the substrate;
a second coating layer having a second polymer covalently bonded to the first polymer on the first coating layer; and
a third coating layer having a drug and a third polymer supporting the drug on the second coating layer, wherein
the second polymer and the third polymer are hydrophobic and form an interpenetrating polymer network structure.

2. The drug-eluting medical device according to claim 1, wherein the second polymer and the third polymer have an identical monomer unit.

3. The drug-eluting medical device according to claim 1 or 2, wherein the third polymer has a lactic acid monomer unit.

4. The drug-eluting medical device according to any one of claims 1 to 3, wherein the second polymer and the third polymer have a lactic acid monomer unit.

5. The drug-eluting medical device according to any one of claims 1 to 4, wherein the second polymer is a crosslinked polymer obtained by crosslinking a base polymer and a crosslinkable monomer.

6. The drug-eluting medical device according to claim 5, wherein
the base polymer has a lactic acid monomer unit, and
the crosslinkable monomer is a (meth)acrylate.

7. The drug-eluting medical device according to claim 6, wherein the (meth)acrylate is a tetra- or higher functional (meth)acrylate.

8. The drug-eluting medical device according to any one of claims 5 to 7, wherein
the third polymer has a lactic acid monomer unit, and
the crosslinkable monomer is contained in an amount of 5 wt% or more and 90 wt% or less with respect to 100 wt% of the base polymer.

9. The drug-eluting medical device according to any one of claims 5 to 7, wherein
the third polymer has a lactic acid monomer unit, and
the crosslinkable monomer is contained in an amount of 30 wt% or more and 90 wt% or less with respect to 100 wt% of the base polymer.

10. The drug-eluting medical device according to any one of claims 5 to 7, wherein
the third polymer has a lactic acid monomer unit, and
the crosslinkable monomer is contained in an amount of 30 wt% or more and 70 wt% or less with respect to 100 wt% of the base polymer.

11. The drug-eluting medical device according to any one of claims 1 to 10, which is a drug-eluting stent.

12. A method for producing a drug-eluting medical device, the method comprising:
a first step of applying a first solution containing a dopamine molecule or an analog thereof as a first coating material onto a substrate and polymerizing the first coating material to form a first coating layer having a first polymer;
a second step of applying a second solution containing a polymer and/or a monomer as a second coating material onto the first coating layer, and then performing heating or light irradiation to form a second coating layer having a second polymer; and
a third step of applying a third solution containing a drug and a polymer as a third coating material onto the second coating layer, and then performing drying to form a third coating layer having a third polymer supporting the drug,
wherein the second polymer and the third polymers are hydrophobic.

13. The method for producing a drug-eluting medical device according to claim 12, wherein
at least a part of the second coating material is soluble in a solvent of the third solution, and
in the third step, the second coating layer swells when the third solution is applied onto the second coating layer.

14. The method for producing a drug-eluting medical device according to claim 12 or 13, wherein the second polymer and the third polymer have an identical monomer unit.

15. The method for producing a drug-eluting medical device according to any one of claims 12 to 14, wherein the third polymer has a lactic acid monomer unit.

16. The method for producing a drug-eluting medical device according to any one of claims 12 to 15, wherein the second polymer and the third polymer have a lactic acid monomer unit.

17. The method for producing a drug-eluting medical device according to any one of claims 12 to 16, wherein the second polymer is a crosslinked polymer obtained by crosslinking a base polymer and a crosslinkable monomer.

18. The method for producing a drug-eluting medical device according to claim 17, wherein
the base polymer has a lactic acid monomer unit, and
the crosslinkable monomer is a (meth)acrylate.

19. The method for producing a drug-eluting medical device according to claim 18, wherein the (meth)acrylate is a tetra- or higher functional (meth)acrylate.

20. The method for producing a drug-eluting medical device according to any one of claims 17 to 19, wherein
the third polymer has a lactic acid monomer unit, and
the crosslinkable monomer is contained in an amount of 5 wt% or more and 90 wt% or less with respect to 100 wt% of the base polymer.

## Patentansprüche

1. Arzneimittel freisetzende medizinische Vorrichtung, die Folgendes umfasst:
ein Substrat,
eine erste Überzugsschicht, die ein erstes Polymer aufweist, das durch Autooxidationspolymerisation eines Dopaminmoleküls oder eines Analogen desselben erhalten wird, auf dem Substrat,
eine zweite Überzugsschicht, die ein zweites Polymer aufweist, das kovalent an das erste Polymer gebunden ist, auf der ersten Überzugsschicht, und
eine dritte Überzugsschicht, die ein Arzneimittel und ein drittes Polymer, welches das Arzneimittel trägt, aufweist, auf der zweiten Überzugsschicht, wobei
das zweite Polymer und das dritte Polymer hydrophob sind und eine sich gegenseitig durchdringende PolymerNetzwerkstruktur bilden.

2. Arzneimittel freisetzende medizinische Vorrichtung nach Anspruch 1, wobei das zweite Polymer und das dritte Polymer eine identische Monomereinheit aufweisen.

3. Arzneimittel freisetzende medizinische Vorrichtung nach Anspruch 1 oder 2, wobei das dritte Polymer eine Milchsäure-Monomereinheit aufweist.

4. Arzneimittel freisetzende medizinische Vorrichtung nach einem der Ansprüche 1 bis 3, wobei das zweite Polymer und das dritte Polymer eine Milchsäure-Monomereinheit aufweisen.

5. Arzneimittel freisetzende medizinische Vorrichtung nach einem der Ansprüche 1 bis 4, wobei das zweite Polymer ein vernetztes Polymer ist, das durch Vernetzen eines Basispolymers und eines vernetzbaren Monomers erhalten wird.

6. Arzneimittel freisetzende medizinische Vorrichtung nach Anspruch 5, wobei
das Basispolymer eine Milchsäure-Monomereinheit aufweist und
das vernetzbare Monomer ein (Meth)acrylat ist.

7. Arzneimittel freisetzende medizinische Vorrichtung nach Anspruch 6, wobei das (Meth)acrylat ein tetra- oder höherfunktionelles (Meth)acrylat ist.

8. Arzneimittel freisetzende medizinische Vorrichtung nach einem der Ansprüche 5 bis 7, wobei
das dritte Polymer eine Milchsäure-Monomereinheit aufweist und
das vernetzbare Monomer in einer Menge von 5 Gew.-% oder mehr und 90 Gew.-% oder weniger in Bezug auf 100 Gew.-% des Basispolymers enthalten ist.

9. Arzneimittel freisetzende medizinische Vorrichtung nach einem der Ansprüche 5 bis 7, wobei
das dritte Polymer eine Milchsäure-Monomereinheit aufweist und
das vernetzbare Monomer in einer Menge von 30 Gew.-% oder mehr und 90 Gew.-% oder weniger in Bezug auf 100 Gew.-% des Basispolymers enthalten ist.

10. Arzneimittel freisetzende medizinische Vorrichtung nach einem der Ansprüche 5 bis 7, wobei
das dritte Polymer eine Milchsäure-Monomereinheit aufweist und
das vernetzbare Monomer in einer Menge von 30 Gew.-% oder mehr und 70 Gew.-% oder weniger in Bezug auf 100 Gew.-% des Basispolymers enthalten ist.

11. Arzneimittel freisetzende medizinische Vorrichtung nach einem der Ansprüche 1 bis 10, die ein Arzneimittel freisetzender Stent ist.

12. Verfahren zum Herstellen einer Arzneimittel freisetzenden medizinischen Vorrichtung, wobei das Verfahren Folgendes umfasst:
einen ersten Schritt des Aufbringens einer ersten Lösung, die ein Dopaminmolekül oder ein Analoges desselben enthält, als ein erstes Überzugsmaterial auf ein Substrat und Polymerisierens des ersten Überzugsmaterials, um eine erste Überzugsschicht zu bilden, die ein erstes Polymer aufweist,
einen zweiten Schritt des Aufbringens einer zweiten Lösung, die ein Polymer und/oder ein Monomer enthält, als ein zweites Überzugsmaterial auf die erste Überzugsschicht und danach Durchführen von Erhitzen oder Lichtbestrahlung, um eine zweite Überzugsschicht zu bilden, die ein zweites Polymer aufweist, und
einen dritten Schritt des Aufbringens einer dritten Lösung, die ein Arzneimittel und ein Polymer enthält, als ein drittes Überzugsmaterial auf die zweite Überzugsschicht und danach Durchführen von Trocknen, um eine dritte Überzugsschicht zu bilden, die ein drittes Polymer, welches das Arzneimittel trägt, aufweist,
wobei das zweite Polymer und die dritten Polymere hydrophob sind.

13. Verfahren zum Herstellen einer Arzneimittel freisetzenden medizinischen Vorrichtung nach Anspruch 12, wobei
zumindest ein Teil des zweiten Überzugsmaterials in einem Lösungsmittel der dritten Lösung lösbar ist und
in dem dritten Schritt die zweite Überzugsschicht quillt, wenn die dritte Lösung auf die zweite Überzugsschicht aufgebracht wird.

14. Verfahren zum Herstellen einer Arzneimittel freisetzenden medizinischen Vorrichtung nach Anspruch 12 oder 13, wobei das zweite Polymer und das dritte Polymer eine identische Monomereinheit aufweisen.

15. Verfahren zum Herstellen einer Arzneimittel freisetzenden medizinischen Vorrichtung nach einem der Ansprüche 12 bis 14, wobei das dritte Polymer eine Milchsäure-Monomereinheit aufweist.

16. Verfahren zum Herstellen einer Arzneimittel freisetzenden medizinischen Vorrichtung nach einem der Ansprüche 12 bis 15, wobei das zweite Polymer und das dritte Polymer eine Milchsäure-Monomereinheit aufweisen.

17. Verfahren zum Herstellen einer Arzneimittel freisetzenden medizinischen Vorrichtung nach einem der Ansprüche 12 bis 16, wobei das zweite Polymer ein vernetztes Polymer ist, das durch Vernetzen eines Basispolymers und eines vernetzbaren Monomers erhalten wird.

18. Verfahren zum Herstellen einer Arzneimittel freisetzenden medizinischen Vorrichtung nach Anspruch 17, wobei
das Basispolymer eine Milchsäure-Monomereinheit aufweist und
das vernetzbare Monomer ein (Meth)acrylat ist.

19. Verfahren zum Herstellen einer Arzneimittel freisetzenden medizinischen Vorrichtung nach Anspruch 18, wobei das (Meth)acrylat ein tetra- oder höherfunktionelles (Meth)acrylat ist.

20. Verfahren zum Herstellen einer Arzneimittel freisetzenden medizinischen Vorrichtung nach einem der Ansprüche 17 bis 19, wobei
das dritte Polymer eine Milchsäure-Monomereinheit aufweist und
das vernetzbare Monomer in einer Menge von 5 Gew.-% oder mehr und 90 Gew.-% oder weniger in Bezug auf 100 Gew.-% des Basispolymers enthalten ist.

## Revendications

1. Dispositif médical à élution de médicament comprenant :
un substrat ;
une première couche de revêtement ayant un premier polymère obtenu par polymérisation par auto-oxydation d'une molécule de dopamine ou d'un analogue de celle-ci sur le substrat ;
une deuxième couche de revêtement ayant un deuxième polymère lié de manière covalente au premier polymère sur la première couche de revêtement ; et
une troisième couche de revêtement ayant un médicament et un troisième polymère supportant le médicament sur la deuxième couche de revêtement, dans lequel
le deuxième polymère et le troisième polymère sont hydrophobes et forment une structure de réseau polymère interpénétrant.

2. Dispositif médical à élution de médicament selon la revendication 1, dans lequel le deuxième polymère et le troisième polymère présentent un motif monomère identique.

3. Dispositif médical à élution de médicament selon la revendication 1 ou 2, dans lequel le troisième polymère présente un motif monomère d'acide lactique.

4. Dispositif médical à élution de médicament selon l'une quelconque des revendications 1 à 3, dans lequel le deuxième polymère et le troisième polymère présentent un motif monomère d'acide lactique.

5. Dispositif médical à élution de médicament selon l'une quelconque des revendications 1 à 4, dans lequel le deuxième polymère est un polymère réticulé obtenu par réticulation d'un polymère de base et d'un monomère réticulable.

6. Dispositif médical à élution de médicament selon la revendication 5, dans lequel
le polymère de base présente un motif monomère d'acide lactique, et
le monomère réticulable est un (méth)acrylate.

7. Dispositif médical à élution de médicament selon la revendication 6, dans lequel le (méth)acrylate est un (méth)acrylate tétrafonctionnel ou de fonctionnalité supérieure.

8. Dispositif médical à élution de médicament selon l'une quelconque des revendications 5 à 7, dans lequel
le troisième polymère présente un motif monomère d'acide lactique, et
le monomère réticulable est contenu en une quantité de 5 % en poids ou plus et de 90 % en poids ou moins rapportée à 100 % en poids du polymère de base.

9. Dispositif médical à élution de médicament selon l'une quelconque des revendications 5 à 7, dans lequel
le troisième polymère présente un motif monomère d'acide lactique, et
le monomère réticulable est contenu en une quantité de 30 % en poids ou plus et de 90 % en poids ou moins rapportée à 100 % en poids du polymère de base.

10. Dispositif médical à élution de médicament selon l'une quelconque des revendications 5 à 7, dans lequel
le troisième polymère présente un motif monomère d'acide lactique, et
le monomère réticulable est contenu en une quantité de 30 % en poids ou plus et de 70 % en poids ou moins rapportée à 100 % en poids du polymère de base.

11. Dispositif médical à élution de médicament selon l'une quelconque des revendications 1 à 10, qui est un stent à élution de médicament.

12. Procédé de production d'un dispositif médical à élution de médicament, le procédé comprenant :
une première étape d'application d'une première solution contenant une molécule de dopamine ou un analogue de celle-ci en tant que premier matériau de revêtement sur un substrat et de polymérisation du premier matériau de revêtement pour former une première couche de revêtement ayant un premier polymère ;
une deuxième étape d'application d'une deuxième solution contenant un polymère et/ou un monomère en tant que deuxième matériau de revêtement sur la première couche de revêtement, puis de réalisation d'un chauffage ou d'une irradiation lumineuse pour former une deuxième couche de revêtement ayant un deuxième polymère ; et
une troisième étape d'application d'une troisième solution contenant un médicament et un polymère en tant que troisième matériau de revêtement sur la deuxième couche de revêtement, puis de réalisation d'un séchage pour former une troisième couche de revêtement ayant un troisième polymère supportant le médicament,
dans lequel le deuxième polymère et le troisième polymère sont hydrophobes.

13. Procédé de production d'un dispositif médical à élution de médicament selon la revendication 12, dans lequel
au moins une partie du deuxième matériau de revêtement est soluble dans un solvant de la troisième solution, et
dans la troisième étape, la deuxième couche de revêtement gonfle lorsque la troisième solution est appliquée sur la deuxième couche de revêtement.

14. Procédé de production d'un dispositif médical à élution de médicament selon la revendication 12 ou 13, dans lequel le deuxième polymère et le troisième polymère présentent un motif monomère identique.

15. Procédé de production d'un dispositif médical à élution de médicament selon l'une quelconque des revendications 12 à 14, dans lequel le troisième polymère présente un motif monomère d'acide lactique.

16. Procédé de production d'un dispositif médical à élution de médicament selon l'une quelconque des revendications 12 à 15, dans lequel le deuxième polymère et le troisième polymère présentent un motif monomère d'acide lactique.

17. Procédé de production d'un dispositif médical à élution de médicament selon l'une quelconque des revendications 12 à 16, dans lequel le deuxième polymère est un polymère réticulé obtenu par réticulation d'un polymère de base et d'un monomère réticulable.

18. Procédé de production d'un dispositif médical à élution de médicament selon la revendication 17, dans lequel
le polymère de base présente un motif monomère d'acide lactique, et
le monomère réticulable est un (méth)acrylate.

19. Procédé de production d'un dispositif médical à élution de médicament selon la revendication 18, dans lequel le (méth)acrylate est un (méth)acrylate tétrafonctionnel ou de fonctionnalité supérieure.

20. Procédé de production d'un dispositif médical à élution de médicament selon l'une quelconque des revendications 17 à 19, dans lequel
le troisième polymère présente un motif monomère d'acide lactique, et
le monomère réticulable est contenu en une quantité de 5 % en poids ou plus et de 90 % en poids ou moins rapportée à 100 % en poids du polymère de base.
